(19) Europäisches Patentamt
European Patent Office
Office européen des brevets

(11) **EP 3 025 265 B1**

(12) **FASCICULE DE BREVET EUROPEEN**

(45) Date de publication et mention
de la délivrance du brevet:
**31.05.2017 Bulletin 2017/22**

(21) Numéro de dépôt: **14759004.6**

(22) Date de dépôt: **23.07.2014**

(51) Int Cl.:
***G06F 19/24*** (2011.01)          ***G06K 9/00*** (2006.01)
***G01N 33/68*** (2006.01)          ***C12Q 1/04*** (2006.01)

(86) Numéro de dépôt international:
**PCT/FR2014/051903**

(87) Numéro de publication internationale:
**WO 2015/011410 (29.01.2015 Gazette 2015/04)**

(54) **MÉTHODE D'ANALYSE D'ÉCHANTILLON BIOLOGIQUE PAR SOUSTRACTION DES AIRES TOTALES RESPECTIVES D'UN SPECTRE DE MASSE D'UN ÉCHANTILLON À ANALYSER ET D'UN SPECTRE DE RÉFÉRENCE**

VERFAHREN ZUR ANALYSE EINER BIOLOGISCHEN PROBE DURCH DIFFERENZBILDUNG DER JEWEILIGEN GESAMTBEREICHE EINES MASSENSPEKTRUMS EINER ZU ANALYSIERENDEN PROBE UND EINES REFERENZSPEKTRUMS

METHOD FOR ANALYSING A BIOLOGICAL SAMPLE BY SUBTRACTION OF THE RESPECTIVE TOTAL AREAS OF A MASS SPECTRUM OF A SAMPLE TO BE ANALYSED AND OF A REFERENCE SPECTRUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.07.2013 FR 1357282**
                **26.09.2013 FR 1359284**

(43) Date de publication de la demande:
**01.06.2016 Bulletin 2016/22**

(73) Titulaires:
• **Université d'Aix Marseille**
  **13284 Marseille Cedex 07 (FR)**
• **Assistance Publique Hôpitaux de Marseille**
  **13005 Marseille (FR)**
• **Fondation Méditerranée Infection**
  **13005 Marseille (FR)**

(72) Inventeurs:
• **RAOULT, Didier**
  **F-13008 Marseille (FR)**
• **CHABRIERE, Eric**
  **F-13009 Marseille (FR)**
• **FLAUDROPS, Christophe**
  **F-13009 Marseille (FR)**
• **BREGEON, Fabienne**
  **F-13821 La Penne sur Huveaune (FR)**

(74) Mandataire: **Domange, Maxime**
  **Cabinet Beau de Lomenie**
  **232 Avenue du Prado**
  **13008 Marseille (FR)**

(56) Documents cités:
**WO-A2-2007/041520     FR-A1- 2 988 843**
**GB-A- 2 467 636        US-A1- 2007 231 921**

**Description**

La présente invention concerne une nouvelle méthode d'analyse d'échantillon biologique à analyser dans laquelle on réalise les étapes successives suivantes:

a) on réalise un spectre de masse de préférence du type MALDI-TOF du dit échantillon à analyser dénommé spectre à analyser, et

b) on réalise au moins un spectre de masse de préférence du type MALDI-TOF d'un échantillon de référence dénommé spectre de référence et

c) on compare ledit spectre à analyser avec un dit spectre de référence.

La spectrométrie de masse est une technique physique d'analyse permettant de détecter et d'identifier des molécules d'intérêt par mesure de leur masse. Son principe réside dans la séparation en phase gazeuse de molécules chargées (ions) en fonction de leur rapport masse/charge (m/Z). Elle utilise un faisceau L.A.S.E.R. pulsé dans l'UV (337 nm) afin de désorber et d'ioniser un mélange de matrice/échantillon co-cristallisé sur une surface métallique. Un excès de matrice évite une trop importante dégradation de l'échantillon, elle absorbe l'énergie et cet apport d'énergie provoque l'expansion en phase gazeuse.

Plus particulièrement, la présente invention concerne l'utilisation de la spectrométrie de masse par désorption-ionisation laser assistée par matrice et temps de vol (en anglais « matrix-assisted laser desorption/ionization time-of-flight mass spectrometry ») dite type MALDI-TOF.

Les applications de la spectrométrie de masse sont très vastes et concernent principalement l'identification de peptides ou de protéines, l'analyse de leur séquence en acides aminés ou encore la mise en évidence de modifications post-traductionnelles. La spectrométrie de masse sous sa variante MALDI-TOF est récemment entrée dans les laboratoires de microbiologie. Elle permet d'identifier et de classifier différents types bactériens en utilisant des bactéries intactes. En effet, parmi les divers composants identifiés dans un spectre de spectrométrie de masse MALDI/TOF de type Biotyper®, certains semblent spécifiques d'une espèce bactérienne précise. Il a ainsi été possible d'identifier un grand nombre d'espèces bactériennes isolées à partir des prélèvements cliniques en utilisant des banques de données élaborées à partir de chacune de ces espèces.

La spectrométrie de masse MALDI-TOF a permis de caractériser différentes spores de champignons, levures et des pollens, notamment dans US 2003/027231.

Enfin, l'analyse MALDI-TOF a été appliquée avec succès pour l'identification de types cellulaires eucaryotes conservés par congélation dans WO 2011/15465. Elle a permis d'étudier la présence de différentes populations lymphocytaires au sein d'un tissu complexe tel que le sang humain et de constituer une banque de données.

La spectrométrie de masse sous sa variante MALDI-TOF (matrix-assisted laser desorption ionization-time-of-flight) permet d'identifier et de classifier différents types bactériens en utilisant des bactéries intactes. En effet, parmi les divers composants identifiés dans un spectre de spectrométrie de masse MALDI-TOF, certains semblent spécifiques d'une espèce bactérienne précise. Ainsi, il a été possible d'identifier grand nombre d'espèces bactériennes dans des prélèvements cliniques en utilisant des banques de données fabriquées à partir de chacune de ces espèces. La spectrométrie de masse de type MALDI-TOF s'impose donc progressivement dans les laboratoires de microbiologie car ses atouts sont nombreux, rapidité et précision d'identification, et faible coût par échantillon.

Parmi les fabricants de spectromètre de masse, deux systèmes MALDI-TOF complets sont disponibles à la vente, commercialisés par les sociétés Bruker Daltonics (Allemagne) et Biomérieux (France). Chaque instrument est accompagné d'un logiciel de pilotage, d'une banque de données et d'un système expert permettant l'identification du micro-organisme reposant sur l'analyse du spectre généré par le spectromètre de masse.

Lorsque l'on compare un nouveau spectre à un autre spectre, comme à l'étape c.), notamment un spectre de référence présent dans la base de données, on met en général en oeuvre le logiciel BIOTYPER® de la société BRUCKER DALTONICS® lequel peut identifier des spectres inconnus par comparaison avec des spectres de référence stockés dans une base de données.

Plus particulièrement, de façon connue encore, on ne prend en compte dans lesdits spectres :

i- les seuls pics dont l'intensité relative, exprimée en pourcentage de l'intensité du pic de plus grande intensité, est supérieure à au moins 3 fois celle du bruit de fond, et

ii- lesdits spectres présentant un score de qualité supérieure à 2, ledit score de qualité étant constitué de l'addition des deux notes n1 et n2 ayant chacune une valeur de 0 à 6 avec :

- n1 est la note attribuée en fonction du nombre de pics caractéristiques dudit spectre n, tel que spécifié plus précisément ci-après, et

- n2 est la note attribuée en fonction de la valeur du rapport signal/bruit (Rmax) du pic de plus grande intensité dudit spectre, tel que spécifié plus précisément ci-après; et

iii- on retient dans le spectre au moins les 50, de préférence au moins les 70 pics de plus grandes intensités.

Les critères ci-dessus sont pris en compte à l'aide notamment du logiciel FLEX CONTROL® et BIOTYPER® RTC développés par le fabricant de spectromètre de masse Bruker Daltonik® GmbH, Leipzig, Germany de référence Microflex LT.

Plus particulièrement encore, les pics desdits spectres sont définis par une paire de coordonnées composées d'une abscisse constituée du rapport masse/charge (m/z) de 2 000 à 20 000 Da, plus particulièrement de 3 000 à 16 000 Da, la coordonnée en ordonnée étant une intensité relative ou en unité arbitraire, et on retient de préférence dans le spectre au moins les 70 pics de plus grandes intensités.

On entend ici par "intensité relative", une intensité exprimée par le rapport de (S/N=intensité du pic/ intensité du bruit de fond).

Les méthodes connues d'analyse spectrale comparative sont donc basés exclusivement sur l'analyse de pics. L'obtention de spectres exploitables par ces méthodes n'est pas toujours possible notamment avec les protocoles classiques d'analyse d'échantillon de milieu complexe à analyser sans isolement préalable de certains constituants.

On entend ici par «spectres exploitables», un spectre qui présente des pics vérifiant notamment les critères d'indice de validation ou de qualité dans les logiciels fournis avec le spectromètre mais aussi et surtout qui révèlent une combinaison de pics dont les positions et intensités soient spécifiques pour identifier ledit échantillon.

La comparaison des spectres selon la méthode de la présente invention peut se faire en combinaison ou complément à une méthode classique avec les logiciels connus commercialisés avec les spectromètres tels que BIOTYPER® qui analyse les spectres pics à pics et sont capables de détecter la présence des pics spécifiques d'un spectre de référence en fonction de la valeur d'un score d'identification comme explicité ci-après dans les exemples de réalisation.

Plus particulièrement, on identifie un genre voire une espèce bactérienne contaminante d'un échantillon à analyser par comparaison avec des spectres de référence de différentes bactéries enregistrées dans la base de données du logiciel Biotyper®. Toutefois, pour certaines bactéries et/ou certaines concentrations, comme rapporté dans les exemples ci-après, il n'est pas toujours possible d'obtenir un score d'identification fiable par rapport à des spectres de référence des bases de données de spectres de bactéries enregistrées dans le dit logiciel Biotyper®.

Le but de la présente invention est donc de fournir une nouvelle méthode d'analyse spectrale comparative qui permette de pallier aux déficiences des logiciels basés sur l'analyse comparative des pics ou au moins pour compléter ces analyses spectrales par pics.

Pour ce faire, selon la présente invention à l'étape c) ci-dessus, on effectue la comparaison des 2 spectres essentiellement en calculant la soustraction entre les aires totales respectives des 2 spectres.

Il s'agit d'une nouvelle méthode d'analyse comparative de spectres avantageuse en ce qu'elle ne considère pas les seuls pics comme le logiciel Biotyper mais la totalité de la courbe du spectre.

On entend ici par «aire totale », la surface entre la courbe du spectre et la ligne de base.

Plus précisément la présente invention fournit une méthode d'analyse d'échantillon biologique à analyser dans laquelle on réalise les étapes successives suivantes:

a) on réalise un spectre de masse de préférence du type MALDI-TOF du dit échantillon à analyser dénommé spectre à analyser, et

b) on réalise au moins un spectre de masse maldi-tof d'un échantillon de référence dénommé spectre de référence, et

c) on compare ledit spectre à analyser avec un dit spectre de référence en calculant la soustraction entre les aires totales respectives du spectre à analyser et du spectre de référence.

La valeur de l'aire totale sous la courbe d'un spectre, à savoir son intégrale correspond à l'intégration de toutes les intensités située entre la courbe du spectre et la ligne de base du spectre (la ligne d'intensité nulle).

Plus particulièrement, à l'étape c), on réalise un spectre de soustraction dans lequel pour chaque valeur de masse, on attribue une intensité résultant de la soustraction des intensités des deux spectres, c'est-à-dire que l'on obtient un spectre avec les pics suivants :

- les pics présents dans l'un des deux spectres, et absent dans l'autre, et

- les pics présents dans les deux spectres mais avec une intensité différente.

Plus particulièrement, à l'étape c), on réalise au moins les étapes successives suivantes :

i.1) on relève les intensités yi de respectivement chacun des points d'abscisse xi correspondant aux valeurs de rapport massique m/z de chaque spectre, et

i.2) pour chaque valeur de xi on calcule une intensité résultante yi3 par soustraction des intensités yi1 et yi2 respectives dudit spectre à analyser (yi1) et respectivement dudit spectre de référence (yi2) :yi3=yi2-yi1, et

i.3) on calcule la somme des intensités résultantes yi3 pour toutes les valeurs xi pour obtenir un score de caractérisation dénommé score $\text{différentiel} = \sum_{i=0}^{i=n} |yi3|$.

Plus particulièrement encore, à l'étape c), on réalise au moins les étapes suivantes :

i.1) on normalise les intensités yi de respectivement chacun des points d'abscisse xi correspondant aux valeurs de rapport massique m/z de chaque spectre en divisant les intensités yi de chaque point d'abscisse xi (m/z) par l'intensité du pic de plus grande intensité, et

i.2) pour chaque valeur de xi on calcule une intensité résultante yi3 normalisée par soustraction des intensités normalisées respectives de deux spectres yi1 et yi2 :yi3=yi2-yi1, et

i.3) on calcule la somme des intensités résultantes normalisées yi3 pour toutes les valeurs xi pour obtenir un score de caractérisation dénommé $\text{score différentiel} = \sum_{i=0}^{i=n} |yi3|$, et

i.4) de préférence, on la normalise en la divisant par la somme des intensités pour toutes les valeurs xi de l'un des spectres, de préférence le spectre de plus petite somme des intensités pour toutes les valeurs xi, pour obtenir un score différentiel normalisé = $\sum_{i=0}^{i=n} |yi3| \sum_{i=0}^{i=n} yip$ avec p=1 ou 2.

Plus particulièrement encore , à l'étape i.3), on réalise les étapes i.3a) et i.3b) de filtrage préalables avant l'étape 3c) suivante :

i.3a) pour chaque valeur de xi, on enlève le bruit de fond en donnant la valeur d'intensité résultante yi3=0 si yi3 est inférieur à un seuil Sy1, de préférence Sy1 étant inférieur ou égal à 10%, de la valeur yi max du pic de plus grande intensité, de préférence Sy1= 5%) de la valeur yi max du pic de plus grande intensité, et

i.3b) on réalise un filtrage en donnant la valeur yi3=0 pour les valeurs de xi pour lesquels à xi' avec xi'-xi inférieur à Sx, Sx étant de préférence inférieur ou égal à 100Da, de préférence 10Da, l'intensité résultante yi3' est de signe opposée à l'intensité résultante yi3 et de valeur absolue d'au moins une valeur seuil relative de Sy2% celle de yi3, de préférence Sy2 étant d'au moins 70% de celle de yi3, notamment du fait d'un décalage des valeurs de xi correspondant aux intensités de deux pics d'un même composant des 2 spectres, et

i.3c) on calcule la somme des intensités restantes après filtrage yi3 pour toutes les valeurs xi pour obtenir un score de caractérisation dénommé $\text{score différentiel} = \sum_{i=0}^{i=n} |yi3|$, et

i.4) de préférence, on la normalise en la divisant par la somme des intensités pour toutes les valeurs xi de l'un des spectres, de préférence le spectre de plus petite somme des intensités pour toutes les valeurs xi, pour obtenir un score différentiel normalisé = $\sum_{i=0}^{i=n} |yi3| \sum_{i=0}^{i=n} yip$ avec p= 1 ou 2.

A l'étape i.3b), ce décalage de pics rapprochés avec changement de signes des intensités peut résulter d'une erreur dans l'acquisition des spectres pour des pics relatifs à un même composant et qui aurait dû être soustrait en l'absence de décalage.
Plus particulièrement encore, le dit échantillon à analyser et le(s) dits échantillon(s) de référence sont issus de prélèvements matières biologiques, de préférence de fluide ou tissu animal ou humain, les dits échantillons de référence et échantillons à analyser pouvant être sains ou pathologiques.

On entend ici par « pathologique», atteint d'une pathologie, notamment une infection, inflammation ou tumeur, dans le cas d'un prélèvement humain ou animal ou pouvant causer une pathologie, notamment une infection microbienne dans le cas d'un fluide biologique humain ou animal, tel que sang ou urine, ou dans le cas d'une matière alimentaire, cosmétique, pharmaceutique ou autre non prélevée d'un individu.

Comme pathologie, on peut aussi citer en particulier, pour un tissu biologique solide, de manière non limitative, une tumeur cancéreuse ou tumeur bénigne non cancéreuse inflammatoire ou non inflammatoire ou encore une fibrose.

Plus particulièrement encore ,selon le type d'échantillons, on peut paramétrer les valeurs d'intensité et les dites valeurs seuils Sx, Sy1 et Sy2 de manière à ce que l'on détermine une valeur ou fourchette de valeurs de score différentiel permettant de déterminer au moins l'identité de nature ou de qualité de l'échantillon à analyser et de l'échantillon de référence, et/ou des valeurs ou fourchette de valeurs de score différentiel de préférence permettant de déterminer le caractère pathologique de l'échantillon à analyser par rapport à un échantillon de même nature mais sain.

Par exemple, on détermine des valeurs v1 à v3, comme suit :

- si le score différentiel est inférieur à une valeur v1, on détermine l'identité des deux échantillons, le procédé étant paramétré pour que v1 soit par exemple inférieur à 100 de préférence inférieur à 10, plus particulièrement v1 étant inférieur à 0.1, et

- si le score différentiel est supérieur à v2 ou compris entre v2 et v3,v2 étant supérieur ou égal à v1 et v3 supérieur à v2, on détermine que ledit échantillon à analyser présente un caractère pathologique, de préférence présentant une pathologie donnée, le procédé étant paramétré pour quev2 et v3 soit inférieur à 100 de préférence inférieur à 10, par exemple de 0.1 à 10.

Plus particulièrement encore, à l'étape b) on réalise au moins un spectre de masse maldi-tof d'un échantillon de référence dénommé spectre de référence qui peut être :

b.1) un spectre de référence obtenu à partir d'un échantillon standard de même nature, notamment de même type cellulaire, provenant d'une autre origine et/ou autre(s) individu(s) que ledit échantillon à analyser, ou

b.2) un spectre de référence d'un échantillon de même nature provenant de la même origine et/ou du même individu que le dit échantillon à analyser.

On entend ici par « même nature», essentiellement de même composition chimique, physique et biologique sans compter des contaminations ou autre pathologie pouvant affecter ledit échantillon à analyser voire ledit échantillon de référence.

Plus particulièrement encore, à l'étape b), on réalise au moins un spectre de masse maldi-tof d'un échantillon de référence dénommé spectre de référence qui peut être :

b.1) un spectre de référence d'un échantillon standard sain de même tissu ou même fluide humain ou animal, ou

b.2) un spectre de référence d'un échantillon sain de même tissu ou même fluide du même individu ; et

- à l'étape c), on détermine si le dit échantillon à analyser est sain ou pathologique, de préférence tumoral et/ou on détermine le type de pathologie, de préférence le type de tumeur selon la valeur de soustraction des aires des deux spectres, de préférence selon la valeur dudit score différentiel.

On entend ici par « tumeur », une pathologie impliquant une prolifération cellulaire de cellules nouvelles d'aspect anormal, entrainant une modification physique locale de la texture dans le cas d'organe solide. Il peut s'agir de tumeur dont les cellules sont cancéreuse c'est-à-dire une tumeur ayant le pouvoir d'envahir les structures voisines comme explicité ci-après ou d'une tumeur non cancéreuse dite « bénigne » ou encore une tumeur d'origine infectieuse ou autre.

Plus particulièrement encore, ledit tissu est fragment d'organe solide.

Les organes peuvent être le poumon (parenchyme pulmonaire, plèvre ou bronches), mais aussi le système colo-digestif (organes creux et glandes annexes, foie, vésicule, pancréas glandes salivaires), ainsi que tout organe de l'organisme (appareil urogénital et reproducteur, muscles, peau, cerveau, glandes endocrines, glandes exocrines, os, ou encore le tissu lymphatique (rate et ganglions), notamment des ganglions de la chaine thoracique.

Plus particulièrement, la présente invention est utile pour réaliser un diagnostic rapide de la qualité d'une ablation de tumeur, notamment dans la chirurgie du cancer.

Plus particulièrement encore, ledit tissu est fragment d'organe solide choisi parmi les fragments de tissu cutané, intestin, notamment oesophage, poumon, bronche, sein, organe de l'appareil uro-génital ou reproducteur masculin ou féminin , notamment prostate, testicule, vessie, utérus, et ganglions.

Plus particulièrement encore, on réalise des étapes b) et c) dans lesquelles :

- à l'étape b), on réalise au moins un spectre de référence établi sur au moins un échantillon de référence de fragment de même tissu sain standard ou provenant du même individu, et

- à l'étape c), on détermine si ledit fragment de tissu à analyser est un fragment de tissu de type non tumoral ou tumoral, de préférence appartenant à une desdites classes de tumeurs c.1 ou c.2 suivantes, selon la valeur de soustraction des aires des deux spectres comparés, de préférence selon la valeur dudit score différentiel :

    c.1- la classe des tumeurs cancéreuses, et

    c.2- la classe des tumeurs non cancéreuses (encore dénommées « tumeurs bénignes »).

Plus particulièrement encore, à l'étape c), on détermine si ledit fragment de tissu à analyser est un fragment de tissu sain ou de type tumoral appartenant au moins à une desdites classes ou sous-classes de tumeurs suivantes, selon la valeur de soustraction des aires des deux spectres comparés, de préférence selon la valeur dudit score différentiel :

    c.1- la classe des tumeurs cancéreuses, et

    c.2.1- la sous-classe de tumeurs non cancéreuses non inflammatoires, et

    c.2.2- la sous-classe des tumeurs non cancéreuses inflammatoires.

Plus particulièrement encore, on paramètre les valeurs d'intensité et les dites valeurs seuils $S_x$, $S_{y1}$ et $S_{y2}$,de manière à ce que l'on détermine des valeurs de scores différentiés $v_1$ à $v_4$, $v_1$ étant inférieur à 100 ,de préférence inférieur ou égal à 10 et $v_2$ à $v_4$ étant entre 0 et 100, de préférence inférieur ou égal à 10, permettant de déterminer l'identité de nature ou de qualité et/ou le caractère pathologique de l'échantillon à analyser de tissu, notamment poumon, et de l'échantillon de référence de même tissu sain, comme suit :

- si le score différentiel entre l'échantillon à analyser et un échantillon de référence de même nature sain est inférieur à une valeur $v_1$, $v_1$ étant de plus particulièrement inférieur à 0.01, notamment $v_1$ étant de 0 à 0,005, l'identité des deux échantillons est certaine, et on conclut à une absence de tumeur (poumon sain),

- si le score différentiel entre l'échantillon à analyser et un échantillon de référence de même nature sain est inférieur à une valeur comprise entre $v_1$ et $v_2$, $v_2$ étant supérieur à $v_1$, notamment le score étant entre 0,005 et 0,1, plus particulièrement entre 0,01 et 0,05, on conclut à la présence d'une tumeur non cancéreuse non inflammatoire,

- si le score différentiel entre l'échantillon à analyser et un échantillon de référence de même nature sain est compris entre $v_2$ et $v_3$, $v_3$ étant supérieur à $v_2$, notamment le score étant compris entre 0,005 et 0,5,plus particulièrement de 0,1 à 0,5, on conclut à la présence d'une tumeur cancéreuse,

- si le score différentiel entre l'échantillon à analyser et un échantillon de référence de même nature sain est supérieure à une valeur $v_3$ ou comprise entre $v_3$ et $v_4$, $v_4$ étant supérieur à $v_3$, notamment le score étant supérieur à 0,5, plus particulièrement supérieur à 1, on conclut à la présence d'une tumeur non cancéreuse inflammatoire.

Plus particulièrement encore, à l'étape c), on détermine si ledit fragment de tissu à analyser est un fragment de tissu sain ou de type tumoral appartenant au moins l'une des sous-classes de tumeurs suivantes choisies parmi:

    c.1.1- la sous-classe des cancers primitifs dudit organe de respectivement différents types cellulaire de cancer,

    c.1.2- la sous-classe des métastases provenant respectivement de différents organes voisins,

    c.2.1- la sous-classe des tumeurs non cancéreuses non inflammatoires, notamment tumeurs non inflammatoires infectieuses (dits « abcès froids ») et tumeurs non inflammatoires non infectieuses telle qu'un nodule, et

    c.2.2- la sous-classe des tumeurs non cancéreuses inflammatoires, notamment des tumeurs inflammatoires infectieuses, et tumeurs inflammatoires non infectieuses.

De préférence, on combine les analyses par le logiciel Biotyper® et logiciel IHU pour améliorer les sensibilités et spécificité de la réponse diagnostique comme explicité plus loin (exemple 4 paragraphe D).

Par exemple, dans le cas de tissu de poumon:

- si la réponse du logiciel Biotyper® permet de déterminer l'absence de tumeur cancéreuse, on conclut à une tumeur non cancéreuse et on ne réalise pas de résection chirurgicale étendue, et

- si la réponse du logiciel Biotyper® permet de déterminer la présence d'une tumeur cancéreuse, on calcule le score différentiel ou score IHU et on conclut à une tumeur cancéreuse si le score différentiel est inférieur à une valeur vo, notamment 0,5 et on conclut à une tumeur non cancéreux inflammatoire si le score différentiel est supérieur à une valeur vo (correspondant à la valeur v3 ci-dessus).

On entend par « fragment de nature tumorale », une tumeur quelconque à savoir: une tumeur cancéreuse aussi bien que non cancéreuse.
On entend par « classe des fragments de tumeur de nature cancéreuse », un cancer primitif ou un cancer de type secondaire, c'est-à-dire de métastases (cellules cancéreuses provenant d'un autre tissu ou organe).
On entend par « sous classe des fragments cancéreux de type de cancer primitif », un cancer de prolifération des cellules d'origine dudit tissu, et ce pour tout type cellulaire du dit organe tel que adénocarcinome, carcinome épidermoïde, carcinome indifférencié, mélanome, sarcome, lymphome et tout autre type de cancer.
On entend par «sous-classe des fragments cancéreux de type de métastase», un cancer provenant de prolifération de cellules provenant d'un autre type d'organe que celui au sein duquel elle est identifiée.
En particulier, lorsque ledit organe est un ganglion, celui-ci est le siège privilégié de métastases provenant d'un organe voisin (proche ou distant) comme indiqué ci-dessus.
On entend par « classe de fragment tumoral de nature non cancéreuse », une tumeur inflammatoire (infectieuse ou non infectieuse), une tumeur non inflammatoire (infectieuse ou non infectieuse).
Dans un mode préféré de réalisation, les organes analysés sont choisis parmi le parenchyme pulmonaire et les ganglions thoraciques régionaux.
Dans un autre mode de réalisation préféré, les échantillons analysés sont choisis parmi les bronches et les ganglions.
Pour les raisons exposées précédemment on analysera attentivement les ganglions à proximité de l'organe principal analysé. En particulier les ganglions régionaux suivants pour les organes voisins suivants :

- pour les poumons, les bronches ou l'oesophage : les ganglions thoraciques, médiastinaux hilaires, para-oesophagiens, abdomino-coeliaques et cervicaux ;

- pour les seins : les ganglions axillaires, thoraciques et cervicaux ;

- pour les viscères intra-abdominaux : les ganglions abdomino-pelviens et sus-claviculaires ;

- pour les organes de la tête et du cou : les ganglions cervicaux et thoraciques ;

- pour la prostate, les testicules et la vessie et autres organes de l'appareil uro-génital et reproducteur féminin ou masculin : les ganglions abdomino-pelviens;

- pour les tissus cutanés et musculaires y compris des membres inférieurs : les ganglions inguinaux, axillaires et/ou du territoire concerné.

On pourra différentier davantage de sous-classes, notamment les sous classes additionnelles suivantes :

- les sous-classes des cancers primitifs dudit organe de respectivement différents types cellulaires de cancer, en particulier pour le poumon les cancers primitifs des différents types listés dans la classification OMS 2004 et 2011 cités ci-après , notamment les types cellulaires de cancers tels que adénocarcinome, carcinome épidermoïde, carcinome indifférencié, mélanome, sarcome, lymphome ; et

- les sous-classes de métastases provenant respectivement de différents autres organes, en particulier pour le poumon, les sous-classes respectives des métastases provenant par exemple de tout cancer localisé dans un autre organe comme par exemple des cancers du sein, du côlon, de la prostate, du pancréas, du rein, de la thyroïde, de l'estomac, des mélanomes, de l'ovaire, des sarcomes.

- les sous-classes de tumeurs non cancéreuses suivantes : tumeur inflammatoire infectieuse, tumeur inflammatoire non infectieuse), tumeur non inflammatoire infectieuse et tumeur non inflammatoire non infectieuse. On peut citer les tumeurs non cancéreuses telles que nodule tuberculeux, sarcoïdose, Histiocytose X, mycose, pneumoconiose, parasitose, granulomatose de Wegener ou toute autre anomalie non cancéreuse.

Plus particulièrement encore, on réalise les étapes a) et b) dans lesquelles:

- à l'étape a), on réalise un dit spectre d'un échantillon à analyser d'une suspension homogène de broyat de dit fragment d'organe comprenant un mélange pluricellulaire et/ou pluritissulaire de différents types de cellules entières non lysées, dilué de préférence dans de l'eau distillée, et

- à l'étape b), on réalise au moins un spectre de référence d'au moins un échantillon de référence de suspension de broyat de respectivement au moins un fragment de référence brut de même tissu d'au moins un individu, le(s) dit échantillon(s) de référence étant préparé(s) de manière identique audit premier échantillon.

La mise en oeuvre d'un dit mélange pluricellulaire et pluritissulaire de cellules entières non lysées signifie que l'on ne réalise pas de traitement d'extraction et/ou séparation de protéines, cellules ou tissus particuliers, et on ne réalise pas de traitement désalinisation ou purification préalable spécifique des protéines ou d'un type cellulaire ou tissulaire particulier.

Plus particulièrement encore, à l'étape a), on réalise une dilution de dit broyat , de préférence dans de l'eau distillée et/ou un solvant de solubilisation de protéines, de préférence encore de l'acide formique et/ou de l'acétonitrile, pour obtenir une dite suspension de broyat d'une concentration de 0,005 à 5 mg/ml, de préférence de 0,01 à 1 mg/ml, ladite suspension de dit broyat analysée étant débarrassée des débris tissulaires de taille supérieure à 100 $\mu$m, de préférence supérieure à 50 $\mu$m, de préférence par centrifugation et récupération du surnageant et sans désalinisation de ladite suspension.

De façon connue, on réalise le broyage d'un fragment d'organe par dilacération ou fragmentation mécanique fournissant des particules de taille inférieure à 100 microns. Ceci peut être réalisé à l'aide d'un dispositif de dispersion/homogénéisation du type à rotor et stator, induisant des forces de cisaillement et de traction et de hautes turbulences dans le fluide notamment un dispositif de broyage du commerce de marques ULTRA TURRAX®ou un dispositif de fragmentation mécanique FastPrep®-24 Instrument (MP biomedicals, FRANCE).

Toutefois, avantageusement, ladite suspension de dit broyat est débarrassée des débris tissulaires de taille supérieure à 100 $\mu$m, de préférence supérieure à 50 $\mu$m, de préférence par centrifugation et récupération du surnageant et sans désalinisation de ladite suspension.

De préférence encore, ladite suspension de broyat comprend un solvant de solubilisation de protéines, de préférence de l'acide formique et/ou de l'acétonitrile.

Plus particulièrement encore, pour éliminer les débris tissulaires de taille supérieure à 100 $\mu$m, de préférence de taille supérieure à 50 $\mu$m, on réalise les étapes suivantes :

- une première centrifugation lente, avec une accélération inférieure à 100 g de ladite suspension de broyat diluée, et

- une deuxième centrifugation plus rapide du surnageant de première centrifugation, à une accélération de plus de 10 000 g, plus particulièrement de 10 000 à 20 000 g, puis

- de préférence, une troisième centrifugation rapide de plus de 10 000 g, de préférence de 10 000 g, du culot de deuxième centrifugation dilué dans de l'eau distillée, et

- récupération dudit culot de deuxième ou, de préférence, troisième centrifugation, que l'on dilue dans des dissolvants de solubilisation de protéines.

Ces échantillons, débarrassés des débris tissulaires grossiers et dilués dans des solvants favorisant la solubilisation des protéines, sont avantageux dans certains modes de réalisation décrits ci-après.

La solubilisation des protéines vise à éviter la coagulation éventuelle des protéines, éventuellement libérées par certaines cellules lors du broyage et/ou dilution décrits ci-dessus.

Le procédé selon la présente invention est simple et rapide à réaliser, n'ayant pas d'étape d'extraction protéique préalable à l'analyse ou autre traitement à réaliser.

Dans un autre mode de réalisation , le dit échantillon à analyser comprend un milieu complexe liquide ou solide contenant des différents types de protéines et/ou différents types de cellules, de préférence des cellules eucaryotes, de préférence des échantillons de fluide biologique humain ou animal contenant des cellules et des macromolécules biologiques, notamment des produits issus du sang ou des urines, et

- à l'étape a), on réalise un dit spectre d'un extrait protéique dudit d'un échantillon à analyser, et

- à l'étape b), on réalise au moins un spectre de référence d'au moins un extrait protéique d'au moins un échantillon de référence de même nature standard ou provenant de la même origine ou même individu que ledit échantillon à analyser, le(s) dit échantillon(s) de référence étant préparé(s) de manière identique audit premier échantillon, et

- à l'étape c), on détermine si le dit échantillon à analyser est sain ou pathologique et/ou on détermine le type de pathologie selon la valeur de soustraction des aires des deux spectres, de préférence selon la valeur dudit score différentiel.

La présente invention permet ainsi l'analyse d'un échantillon biologique liquide complexe contenant des cellules tel qu'un échantillon de produit sanguin issu du sang notamment un concentré de plaquettes comme décrit ci-après ou les urines, pour une détection d'agents microbiens dans ce milieu complexe par spectrométrie Maldi-Tof sans étape d'isolement dans un délai de pas plus de 24h.

Les produits de transfusion du sang aussi dénommés « produits sanguins labiles» («PSL») comprennent les concentrés de globules rouges, les concentrés de plaquettes, plasma frais congelé ; et des médicaments préparés industriellement à partir de pools de centaines de plasma (albumine, immunoglobulines intraveineuses, facteurs de la coagulation).

Des liquides de concentrés de plaquettes de ce type sous l'appellation ci-après de «concentré plaquettaire» ou «CP», sont connus et commercialisés avec des concentrations d'au moins 300.109 plaquettes/l dans un soluté nutritif à base de sucre et de sel. Ils se conservent 5 jours à 20-24°C et sous agitation constante.

Plus particulièrement, ledit extrait protéique de dit échantillon à analyser et le dit échantillon de référence sont préparés par un procédé comprenant les étapes suivantes dans lesquelles :

a.1) on réalise une lyse des cellules dudit échantillon, puis

a.2) on réalise une extraction des protéines entières et solubilisées à partir d'un lysat dudit échantillon après culture, de l'étape a.1), et

b) on réalise un spectre par spectrométrie de masse du type MALDI-TOF dudit extrait protéique obtenu à l'étape a.2), et

c) on détermine si ledit échantillon à analyser est sain ou pathologique par comparaison du spectre obtenu à l'étape b) avec au moins un spectre de référence d'un extrait protéique de référence d'un échantillon standard de même nature que ledit échantillon à analyser ayant subi le même traitement de lyse et extraction des étapes a.1) et a.2).

Plus particulièrement, aux étapes a.1) et a.2), ledit extrait protéique est obtenu en effectuant les étapes successives suivantes :

1) on élimine le liquide du dit échantillon à analyser, et on récupère un premier résidu solide, et

2) de préférence, on ajoute une solution de détergent au dit premier résidu solide, et

3) on élimine le liquide à nouveau et on récupère un deuxième résidu solide, et

4) on réalise une lyse des cellules présente dans ledit deuxième résidu solide, de préférence une lyse chimique, et

5) on solubilise les protéines présentes dans le dit deuxième résidu solide, de préférence avec une solution de solvant, et

6) on élimine les substances non solubilisées, pour récupérer une solution essentiellement constituée de protéines entières.

A l'étape 2), on supprime les interactions entre les dits microbes et les autres composants contenus dans ledit échantillon avec la dite solution de détergent ajoutée au dit premier résidu solide.

Plus particulièrement encore, pour traiter un échantillon de concentré de plaquettes, ledit extrait protéique est obtenu en effectuant les étapes successives suivantes :

1) on élimine le liquide du dit échantillon à analyser, en réalisant une première centrifugation et éliminant le surnageant de centrifugation, et en récupérant un premier résidu solide qui est le culot de première centrifugation, et

2) on ajoute une solution de détergent au dit premier résidu solide, de préférence une solution de saponine, et

3) on récupère un deuxième résidu solide par une deuxième centrifugation et en éliminant le surnageant et en récupérant un deuxième résidu solide qui est le culot de deuxième centrifugation, et

4) on réalise une lyse chimique des cellules présente dans ledit deuxième résidu solide, de préférence on réalise un traitement chimique avec une solution acide d'acide formique, et

5) on solubilise les protéines présentes dans le dit deuxième résidu solide de lysat cellulaire, de préférence avec une solution de solvant acétonitrile et

6) on élimine les substances non solubilisées, par une troisième centrifugation et en éliminant le culot et récupérant le surnageant de troisième centrifugation, pour récupérer une solution essentiellement constituée de protéines entières.

Plus particulièrement encore, ledit spectre de référence spécifique de dit extrait de concentré de plaquettes sanguines non contaminé comprend au moins les pics caractéristiques de plus grande intensités, dans le tableau A suivant dans lequel les différents pics caractéristiques sont caractérisés par leurs coordonnées en abscisse m/z (Da) et leurs coordonnées en ordonnée d'intensité relative exprimées en S/N (intensité du pic/intensité du bruit de fond), les valeurs des masses moléculaires m/z pouvant varier jusqu'à ±1 Da de la valeur moyenne mentionnée dans le tableau A, et les valeurs d'intensité relative pouvant varier jusqu'à ±20% de la valeur moyenne d'intensité relative mentionnée :

TABLEAU A :

| Index | m/z | Intensité (S/N) | Index | m/z | Intensité (S/N) |
|---|---|---|---|---|---|
| 2 | 2039,33 | 0,82 | 45 | 5941,47 | 12,96 |
| 3 | 2053,09 | 0,96 | 46 | 6123,11 | 4,61 |
| 4 | 2172,47 | 2,81 | 47 | 6195,2 | 4,99 |
| 5 | 2188,26 | 2,08 | 48 | 6253,84 | 12,31 |
| 6 | 2206,14 | 1,49 | 49 | 6329,64 | 34,15 |
| 7 | 2335,49 | 0,81 | 50 | 6399,07 | 4,69 |
| 8 | 2587,08 | 9,77 | 51 | 6437,89 | 19,1 |
| 9 | 2677,66 | 6,53 | 52 | 6636,41 | 10,92 |
| 10 | 2824,37 | 44,11 | 53 | 6667,28 | 30,17 |
| 11 | 3124,2 | 2,88 | 54 | 6725,81 | 41,44 |
| 12 | 3170,04 | 2,43 | 55 | 7193,2 | 13,26 |
| 13 | 3332,51 | 4,02 | 56 | 7307,12 | 1,57 |
| 14 | 3372,52 | 27,37 | 57 | 7355,33 | 2,03 |
| 15 | 3443,5 | 48,02 | 58 | 7437,27 | 19,85 |
| 16 | 3487,23 | 15,79 | 59 | 7567,73 | 42,56 |
| 17 | 3717,3 | 2,85 | 60 | 7642,27 | 28,22 |
| 18 | 3783,47 | 6,75 | 61 | 7768,13 | 745,58 |

(suite)

| Index | m/z | Intensité (S/N) | Index | m/z | Intensité (S/N) |
|---|---|---|---|---|---|
| 19 | 3820,02 | 5,97 | 62 | 7839,16 | 152,91 |
| 20 | 3883,82 | 343,89 | 63 | 7924,93 | 114,32 |
| 21 | 3920,17 | 34,15 | 64 | 8022,69 | 21,31 |
| 22 | 3963,03 | 32,32 | 65 | 8144,94 | 177,92 |
| 23 | 4049,38 | 1,6 | 66 | 8211,76 | 12,77 |
| 24 | 4072,05 | 48,19 | 67 | 8584,21 | 37,68 |
| 25 | 4292,54 | 15,29 | 68 | 8652,46 | 28,04 |
| 26 | 4326,29 | 5,36 | 69 | 8719,83 | 16,44 |
| 27 | 4481,88 | 47,61 | 70 | 8963,52 | 106,86 |
| 28 | 4568,92 | 4,21 | 71 | 9039,83 | 6,86 |
| 29 | 4623,37 | 14,23 | 72 | 9136,49 | 7,45 |
| 30 | 4646,43 | 14,15 | 73 | 9249,43 | 26,67 |
| 31 | 4688,05 | 31,78 | 74 | 9291,19 | 35,04 |
| 32 | 4732,37 | 17,33 | 75 | 9375,12 | 58,79 |
| 33 | 4760,03 | 62,14 | 76 | 9464,76 | 35,82 |
| 34 | 4813,43 | 38,33 | 77 | 9519,48 | 89,66 |
| 35 | 4896,95 | 40,58 | 78 | 9621,13 | 71,71 |
| 36 | 4966,02 | 17,88 | 79 | 10102,91 | 69,65 |
| 37 | 5052,1 | 44,86 | 80 | 10189,05 | -0,33 |
| 38 | 5137,16 | 4,66 | 81 | 10262,85 | 2,29 |
| 39 | 5268,85 | 46,78 | 82 | 10617,08 | 29,7 |
| 40 | 5310,04 | 31,23 | 83 | 10701,45 | 1,97 |
| 41 | 5360,04 | 138,98 | 84 | 10843,44 | 40,58 |
| 42 | 5422,73 | 19,14 | 85 | 12392,91 | 2,15 |
| 43 | 5652,14 | 229,61 | 86 | 12503,41 | 9,72 |
| 44 | 5698,21 | 13,9 | 87 | 13441,97 | 3,28 |

On fait état de la valeur m/z, z représentant la charge électrique de l'espèce ionisée. Mais, en pratique, m/z peut être assimilé à la masse moléculaire m, avec z=1, car la quantité de protéines qui s'ionisent 2 fois (z=+2), voire 3 fois (z=+3), est négligeable, compte-tenu de la limite de sensibilité des techniques de mesures.

Les valeurs rapportées ont été obtenues par une analyse de l'extrait protéique de CP volume à volume, avec une solution de matrice d'acide $\alpha$-cyano-4-hydroxycinnamique, lors de ladite analyse par spectrométrie de masse et pas avant.

La présente invention a également pour objet une méthode caractérisée en ce qu'on met en oeuvre un programme d'ordinateur comportant au moins les instructions pour l'exécution des étapes i.1) à i.3) du procédé de traitement de spectres de préférence un support d'enregistrement lisible par un ordinateur sur lequel est enregistré le dit programme d'ordinateur.

Les différentes étapes du procédé selon l'invention sont déterminées par des instructions de programmes d'ordinateurs et ou d'automate programmable industriel (API) avec interface homme machine (IHM).

En conséquence, l'invention vise aussi un programme d'ordinateur sur un support d'informations, ce programme étant susceptible d'être mis en oeuvre dans un module de commande, ce programme comportant des instructions adaptées à la mise en oeuvre des étapes du procédé selon l'invention.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

D'autres caractéristiques et avantages de la présente invention ressortiront mieux à la lecture de la description qui va suivre, faite de manière illustrative et non limitative, en référence aux dessins annexés sur lesquels :

La figure 1 représente plusieurs spectres de spectrométrie de masse maldi-tof de tissus de poumon sain de l'exemple 4.

Les figures 2A à 2C représentent des spectres de spectrométrie de masse maldi-tof traités selon le logiciel IHU d'échantillons de tissu de poumon sain et tissu de poumon tumoral non cancéreux inflammatoire commentées à l'exemple 4.

Les figures 3A à 3C représentent des spectres de spectrométrie de masse maldi-tof traités selon le logiciel IHU d'échantillons de tissu de poumon sain et tissu de poumon cancéreux commentées à l'exemple 4.

La figure 4 illustre la dispersion des scores différentiels calculés avec le logiciel IHU pour différentes sous-classes d'échantillons de poumons sains(A), cancéreux (B), tumeurs bénignes non inflammatoires (C), tumeur bénigne inflammatoires (D) représentée selonla représentation connue de l'homme de l'art dite des « boites à moustaches » ou « Box Plots » commentée à l'exemple 4.

Les figures 5A à 5C et 6A à 6C commentées à l'exemple 7représentent des spectres de spectrométrie de masse maldi-tof traités selon le logiciel IHU d'extraits protéiques d'échantillon standard de concentré de plaquettes non contaminé et échantillon de concentré de plaquettes contaminés par *E.coli* (figures 5A à 5C) et par *S.aureus* (figures 6A à 6C).

La figure 7 représente différents spectres d'extrait protéique de concentrés de plaquettes, non contaminés (spectres 4a et 4b), contaminés par différentes bactéries *S. aureus (4c), E. coli (4d), P. stuartii (4e), P. aeruginosa (4f)*.

**Exemple 1 :** Protocole de préparation des fragments tissulaires de poumon.

1) Prélèvement du tissu

Les résections pulmonaires ont été effectuées selon la technique chirurgicale standard. La pièce de résection encore appelée fragment de tissu, a été le siège de 4 biopsies au coeur de la tumeur visible et quatre autres fragments prélevés le plus à distance possible de la tumeur en territoire supposé sain. Un exemplaire de chaque territoire a été adressé pour l'examen histologique extemporané, un autre exemplaire était destiné à l'examen histologique définitif, un autre était adressé pour conservation à la tumoro-thèque, le dernier était destiné aux traitements selon l'invention et études par spectrométrie de masse dans sa variante MALDI-TOF.

2) Préparation des échantillons en vue de l'analyse par spectrométrie MALDI-TOF

Le recueil de matériel a été effectué après broyage du parenchyme dilué dans de l'eau distillée à l'aide du broyeur du type disperseur/homogénéiseur Ultra-turrax® de référence T25 digital de vitesse variable de 3 400 à 24 000 tours par minute (ci-après "tr/min" et de puissance électrique de 500 à 300 W (high-performance disperser), selon la technique décrite dans le protocole résumé ci-dessous. Le broyat a ensuite été utilisé tel quel et déposé sur la plaque du spectromètre MALDI TOF ou à titre comparatif a subi une phase de purification selon différents modalités notamment tel que décrit ci-après en reprenant le protocole de purification/solubilisation simple déjà utilisée pour l'identification des bactéries dans des urines au sein du laboratoire de microbiologie clinique de l'hôpital de la Timone (Marseille, France).

2.1) Traitement préalable du fragment pulmonaire (fragmentation et broyage)

On a réalisé le protocole suivant comprenant les étapes suivantes de :

1- Division du fragment initial de poumon en 10 portions sensiblement égales,

2- Echantillonnage d'un fragment avec sa pesée pour un travail sur un fragment de 0.08 à 0.3 g sur cette série,

3- Nettoyage du broyeur : par mise en marche de la rotation et plongée de l'outil de dispersion dans un tube d'eau stérile le couvrant jusqu'en haut puis répétition de cette manoeuvre dans de l'alcool et répétition encore dans de l'eau distillée,

4- Pesée du fragment et mise en place dans un tube stérile sec,

5- Adjonction d'un volume d'eau distillée égal à 10 fois le poids du fragment de poumon réalisant ainsi la première dilution à 1/10ème. Maintien du tube contenant le fragment et l'eau dans de la glace pilée pour éviter tout échauffement lors du broyage, ou lorsque le poids de l'échantillon est inférieur à 0.01g, une dilution est réalisée d'emblée dans 1,6 ml d'eau distillée afin de réaliser une dilution à 1/160,

6- Broyage par mise en marche progressive de la rotation jusqu'à 2400 tr/min pendant 1 minute correspondant au temps habituellement nécessaire pour obtenir une suspension homogène,

7- Prélèvement d'une partie de la suspension pour mise en réserve. Ajout d'un volume d'eau distillée à la suspension restante, ajusté au fur et à mesure des résultats (de 1/10ème à 1/160ème) jusqu'à ce que l'on se fixe le facteur optimal qui s'est avéré être 1/160 pour obtenir une suspension aqueuse de broyat homogène trouble de concentration de 0,0005 à 0.001mg/ml à l'issue de l'étape de broyage,

8- Pour certains échantillons : conservation du broyat en plusieurs aliquots entreposés à -80°C,

9- Au moment de l'analyse, dépôt de suspension de de broyat, le cas échéant décongelé sur la plaque du MALDI-TOF (10 dépôts de 1 microlitre par échantillon pour la mise au point du protocole).

2.2) Méthode de séparation des débris cellulaires grossiers de taille supérieure à 100 $\mu$m et solubilisation de protéine

On a réalisé le protocole suivant comprenant les étapes suivantes consistant à :

1- Centrifuger la suspension de broyat diluée dans de l'eau, obtenue à l'étape 7- de la méthode 2.1) ci-dessus, pendant 5 minutes à 20 g (soit 500 tr/min avec la centrifugeuse de la société THERMOSCIENTIFIC® de référence HERAUS PICO 17),

2- Prélever le surnageant, le centrifuger pendant 5 minutes à 16 500 g (soit 14 000 tr/min avec la centrifugeuse ci-dessus),

3- Rejeter le surnageant, ajouter 1 ml d'eau distillée et remettre le culot en suspension,

4- Agiter (Vortexer) puis centrifuger 5 min à 16 500 g,

5- Rejeter le surnageant et récupérer le culot,

6- Ajouter au culot lavé 5 $\mu$l d'acide formique 100%. Bien mélanger avec la pipette et attendre 5 min,

7- Ajouter 5 $\mu$l d'acétonitrile à 100%. Bien mélanger,

8- Centrifuger 2 minutes à 16 500 g,

9- Récupérer le surnageant et déposer le surnageant sur la plaque du spectromètre de masse MALDI-TOF (10 spots par échantillons).

**Exemple 2 :** Réalisation des spectres par spectrométrie de type MALDI-TOF sur des échantillons de broyats de poumon.

L'échantillon est co-cristallisé avec une matrice de type hcca (acide 4-hydroxy alpha-cyano cinnamique) puis les spectres seront obtenus sur l'appareil Microflex LT® (Bruker Daltonics) selon la méthodologie suivante :

Le protocole de dépôt comprend les étapes suivantes consistant à:

1- déposer 1 $\mu$L d'extraction pulmonaire sur une position prédéfinie (cercles appelés « spots ») de la cible MICROFLEX (plaque en acier inoxydable BRUKER DALTONICS,

2- déposer 1 μL de matrice (HCCA à saturation dans 50% d'acetonitrile 2,5% d'acide trifluoroacétique, qsp eau distillée),

3- laisser sécher pendant 5 minutes à température ambiante,

4- introduire la plaque (cible Microflex® modèle de référence 224989 dénommé MSP 96) contenant les dépôts dans le spectromètre de masse Microflex® LT,

5- Démarrer l'ordinateur associé au spectromètre pour lancer les analyses, et

6- Lancer l'acquisition des spectres de masse MALDI-TOF.

Les paramètres appliqués au spectromètre de masse sont les suivants :

- Mode linéaire positif

- Electrode IS1 : 20,00 kV, Electrode IS2 : 16,65 kV, Electrode de focalisation : 7,20 kV

- Post-ion-extraction (PIE) : 120 ns, Fréquence du laser : 60Hz, Gain du détecteur : 8,4 X, Gamme de masse : de 2 000 à 20 000 Da.

Les spectres sont obtenus via une méthode automatique contrôlée par le logiciel FLEXCONTROL® du fabricant BRUKER DALTONICS® les paramètres sont les suivants pour chaque spot :

- Somme de 240 tirs par série de 40 tirs

- Afin que les tirs aient balayé l'ensemble de l'échantillon de façon homogène l'acquisition se fait sur 6 positions différentes selon une géométrie hexagonale.

- Pré-tirs : 10 tirs à 40% de la puissance maximum laser servent à dessaler l'échantillon (contamination par les sels minéraux)

- Puissance laser : 30 % à 45 % régulée via le logiciel FLEXCONTROL®

- Sélection du spectre : sur une gamme de masse de 4 000 à 10 000 Da.

- L'acquisition des spectres est au final réalisée via le logiciel Biotyper-RTC® du fabricant BRUKER DALTONICS® qui permet d'appliquer la méthode automatique décrite ci-dessus à une série d'échantillons en prenant en compte les critères mentionnés précédemment et un score de qualité ou score de validation qui représentent l'addition de 2 notes ni + n2 (ni étant fonction du nombre de pics et n2 fonction du rapport signal/bruit de fond).

- ni est la note attribuée en fonction du nombre de pics caractéristiques du spectre n, avec

  . ni = 0 pour $n \leq 8$,. ni = 1 pour $8 < n \leq 27$,. ni = 2 pour $27 < n \leq 43$,. ni = 3 pour $43 < n \leq 78$,. ni = 4 pour $78 < n \leq 86$,. ni = 5 pour $86 < n \leq 110$,. ni = 6 pour $110 < n$, et

- n2 est la note attribuée en fonction de la valeur du rapport signal/bruit (Rmax) du pic de plus grande intensité du spectre, avec

  . n2 = 0 pour $Rmax \leq 6,8$,. n2 = 1 pour $6,8 < Rmax \leq 26,8$,. n2 = 2 pour $26,8 < Rmax \leq 52,6$,. n2 = 3 pour $52,6 < Rmax \leq 208,6$,. n2 = 4 pour $208,2 < Rmax \leq 398,6$,. n2 = 5 pour $398,6 < Rmax \leq 1\ 092,2$,. n2 = 6 pour $1\ 092,2 < Rmax$.

Un spectre est pris en compte si son score de validation (n1+n2) est supérieur à 2.

D'autre part, le logiciel BIOTYPER® établit un spectre moyen à partir d'au moins 4, de préférence au moins 10, spectres réalisés sur 10 dépôts sur plaque d'un même échantillon.

Ledit spectre moyen issu de plusieurs spectres est établi par le logiciel BIOTYPER® à partir des spectres individuels dans lesquels sont recherchés des pics dits « valides » selon une méthode se résumant ainsi :

- l'étendue du pic sur l'axe des abscisses ne doit pas excéder 25 Da,

- la variation de la ligne de base du pic doit être contenue dans l'intervalle moyen de variation des lignes de base de l'ensemble des pics du spectre, et

- l'intensité du pic est considérée lorsque le rapport signal/bruit de fond est supérieur à 3.

Une fois les pics d'intérêt ainsi sélectionnés, le logiciel réalise une moyenne des intensités pour chaque valeur de m/z présents dans au moins 25% des spectres individuels.

Exemple 3 : Analyse des spectres de broyats de poumons.

A) Les inventeurs ont comparé entre eux deux spectres moyens entre eux ou un nouveau spectre avec les spectres moyens contenus dans la base de données de spectres d'échantillons connus décrits ci-après grâce au logiciel BIOTYPER-RTC® développé par le fabricant du spectromètre de masse MALDI-TOF (BRUKER DALTONICS®).

Le logiciel BioTyper® identifie des spectres inconnus par comparaison avec des spectres de référence stockés dans une base de données ou autre spectre comparatif par une analyse des pics qu'ils contiennent. Les résultats d'identification sont donnés sous forme de d'un score d'identification calculé comme ci-après explicité.
Dans un premier temps, les deux spectres sont alignés. On assigne une erreur acceptable pour la masse des pics de chaque spectre ($\pm$1 Da) et le logiciel repositionne les deux spectres (autrement dit, si l'écart entre les pics du même spectre et entre les pics de l'autre spectre est trop différent, le logiciel ne pourra pas aligner les spectres).
Ensuite, les spectres obtenus subissent le traitement suivant par le logiciel BioTyper® 3.0:

- Une soustraction de ligne de base, afin d'éliminer le bruit de fond, et

- Un lissage des pics par mise à 0 des intensités inférieures à un seuil égal à 10% de l'intensité du pic maximale, afin d'augmenter la résolution, puis

- une liste de masse de pics est créée.

Ensuite, la comparaison d'un spectre inconnu avec le spectre de référence (par exemple, le spectre moyen présent dans la base de données) est évaluée en utilisant des scores d'identification dédiés. Les algorithmes, qui permettent de calculer ces scores, sont décrits dans le manuel fourni par Bruker Daltonics®.
En résumé, le score d'identification est égal à log (indice1 + incide2 + indice3), avec :

- un indice 1 de valeur maximale de 1000 quand l'ensemble des pics de la référence de la base de données sont trouvés dans le spectre à identifier, l'indice 1 étant de zéro si il n'y a aucune correspondance, et

- un indice 2 étant de valeur maximale de 1000, si l'ordre de classification des pics par intensité croissante du spectre à identifier est identique à l'ordre de classification des pics de la référence, et

- un indice 3 étant de valeur maximale de 1000, si les pics non attribués du spectre à identifier ne matche sur aucune référence.

Les algorithmes, qui permettent de calculer ces scores, sont décrits dans le manuel fourni par BRUKER DALTONICS® qui donne un exemple succinct de la méthode employée dans son manuel "Software for microorganism identification and classification". Ainsi, un pic identique entre deux spectres ou avec un spectre de la base de données et le spectre analysé, obtient un score positif alors que l'absence d'un pic se traduit par un score négatif. L'ensemble des scores obtenus est exprimé en échelle logarithmique. Les scores obtenus pour identifier et classifier les microorganismes (Fournier PE, Couderc C, Buffet S, Flaudrops C, Raoult D. Rapid and cost-effective identification of Bartonella species using mass spectrometry. J Med Microbiol. 2009, 58:1154-1159) sont ceux que les inventeurs ont utilisés pour identifier et classifier les types de tissus solides cancéreux et non cancéreux.
Ainsi, un score d'identification compris entre 0 et 1,699 permet d'exclure l'identité du spectre analysé avec celui d'un type donné présent dans la base de données ou le spectre avec lequel il est comparé. Un score d'identification compris entre 1,700 et 1,999 suggère que le spectre analysé est identique à celui présent dans la base de données et un score d'identification supérieur à 2,0 permet une identification certaine.

B) Essais réalisés sur des broyats de poumons.

1) Dans une série d'expérimentations, il a été observé que la signature spectrale obtenue en spectrométrie de masse type MALDI-TOF sur un broyat de poumon dilué était spécifique d'espèce pour le rat, la souris et le mouton qui étaient les 3 espèces testées avec des pics discriminants dans la zone spectrale correspondant aux masses situées entre 7 000 et 8 500 D. p<0,001 (spectres non représentés). Il a été testé les poumons de plusieurs espèces de mammifères (rat, souris, mouton) y compris l'humain. Ceci a confirmé la spécificité d'espèce.

A ce stade, on a noté une différence sur des échantillons humains issus d'un même patient. La recherche a posteriori de renseignements cliniques et des résultats histologiques a révélé qu'il s'agissait d'un fragment pulmonaire porteur d'une tumeur cancéreuse. L'idée est alors apparue que l'analyse rapide par spectrométrie de masse de type MALDI-TOF non précédé d'une désalinisation ni d'extraction et purification des protéines pouvait permettre de détecter la présence de cellules cancéreuse dans un fragment de poumon et différencier ainsi un fragment sain d'un fragment tumoral.

2) Dans une autre série d'expérimentations, on a analysé 32 paires de broyats d'échantillons de poumon dilués à environ 0,625 mg/ml dans l'eau distillée issus de fragments de poumons humains prélevés au cours d'une chirurgie thoracique pour tumeur cancéreuses du poumon. Les fragments étaient codés sans révéler lesquels (au nombre de 32) étaient prélevés au sein de la tumeur ni ceux (32) en zones saines.

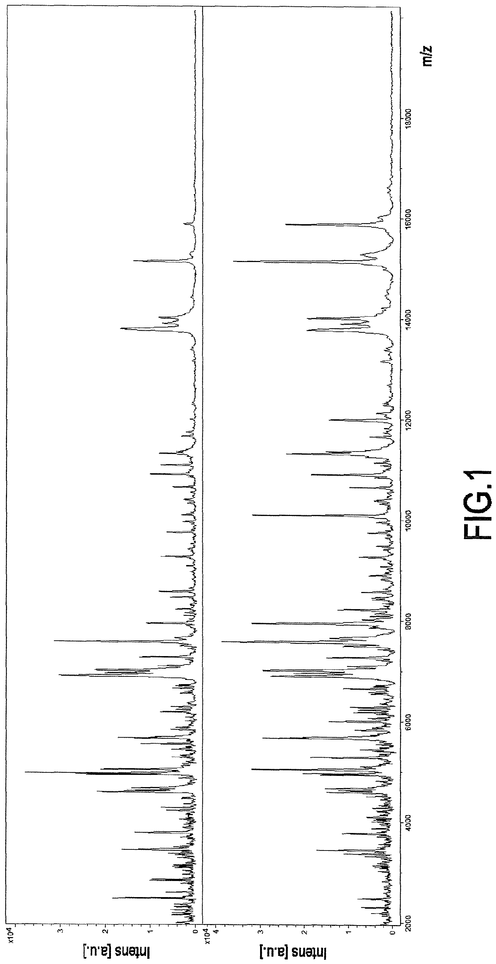

Les spectres des tissus de poumons sains représentent un profil reproductible (figure 1).

Les résultats bruts ont permis d'observer une séparation des profils spectraux en deux principaux groupes alors que 2 échantillons se rangeaient ailleurs.

Plus précisément, un numéro de 1 à 32 était attribué à chaque fragment suivi du numéro 1 ou 2 selon qu'il était à priori prélevé en zone non tumorale (1) ou tumorale (2). Ainsi, une liste de 64 échantillons a été constituée.

Lorsqu'on compare l'ensemble des spectres issus de ces 64 + 2 = 66 échantillons préparés et analysés selon la même méthode, on observe une séparation des profils spectraux en deux principaux groupes séparés par une distance témoignant d'une différence significative sur un dendrogramme (non représenté).

La levée de l'inconnue diagnostique a été réalisée a posteriori révélant un fragment cancéreux et un fragment sain pour la plupart des sujets et l'absence de cancer pour 3 sujets (diagnostic définitif = 1pseudo-tumeur inflammatoire et 2 pneumopathies infectieuses).Dans tous les cas on a observé que les deux spectres Non cancéreux et Cancéreux pour chaque patient étaient distincts.

Ces résultats ont conduit à rechercher un système performant permettant de classer un fragment tissulaire en tant que tissu cancéreux ou non cancéreux avec une sensibilité et une spécificité compatible avec une application clinique en tant qu'outil diagnostique ultra-rapide pour aide à la décision lors des exérèses chirurgicales.

Les spectres issus des analyses successives effectuées à partir des 32 paires d'échantillons cancer/non-cancer appariés à partir d'un même patient (n = 32 patients) ont été examinés. Les exigences qualitatives des spectres et le besoin d'en avoir suffisamment pour la base et pour le test expliquent que l'on n'ait pu travailler que sur les spectres de 23 des 32 échantillons tumoraux et de 30 des 34 (32+ 2 issus de malades à poumons sains) échantillons non tumoraux, ce qui correspond à environ 200 spectres dans chaque base de données (200 pour la base poumon tumoral et 200 pour la base poumon non tumoral).

A partir des 53 (23+30) échantillons, 53 spectres moyens de référence (SM) ont été créés. Le résultat de la confrontation informatique à ces banques de données, a été rendu sous la forme d'un diagnostic tumeur/ non tumeur dont la pertinence est appréciée par un test statistique exprimé par un score d'identification de 0 à 3. Cette valeur statistique est définie par le fabricant via un algorithme spécifique à Brucker Daltonics®. Pour une valeur comprise entre 0 et 1,699, le spectre, donc l'échantillon dont il est issu, est exclu de toute similitude avec ceux de la base de données et rendu comme étant « inclassable ». Un score compris entre 1,700 et 1,999 suggère une similitude de degré moyen, un score supérieur à 2,0 permet une classification certaine comme appartenant au groupe trouvé dans la base de données. Sur ces critères, les spectres testés en aveugle ont été classées correctement pour 13/23 échantillons tumoraux et 18/30 échantillons non tumoraux seulement.

Par cette méthode classification par le modèle Biotyper®, il est donc possible de déterminer si le tissu est sain ou cancéreux avec une prédiction relativement bonne mais insuffisante.

Dans une autre série de 196 autres échantillons traités en temps réel au bloc opératoire une base de données initiale de 20 échantillons issus de 10 paires sain et cancéreux a été constituée et incrémentée au fur et à mesure en vue d'une classification de chaque échantillon nouveau à l'aide du logiciel Biotyper. Ainsi, chaque échantillon nouveau était testé en aveugle et comparé à la base de donnée complétée des échantillons précédents. Compte tenu que la recherche d'un spectre similaire avec le logiciel Biotyper® est réalisée pour chaque spot déposé, ici 4 spots par échantillon, et compte tenu que le logiciel fourni jusqu'à 10 niveaux de réponses en fonction des en analogies retrouvées avec les spectres de référence de la base de donnée, n'ont été prises compte que les 2 premières réponses pour chaque spot, ce qui constitue 8 réponses pour chaque échantillon. Pour le diagnostic avec Biotyper®, la classe pour laquelle au moins 5 réponses sur 8 étaient similaires a été considérée. Ainsi, si 5 réponses sur 8 ou plus donnaient une analogie avec un fragment sain de la base de données, le fragment à analyser était considéré sain. Si 5 réponses sur 8 ou plus donnaient une analogie avec un fragment cancéreux (ou tumoral bénin ou malin, ou tumoral bénin selon la base de donnée choisie), le fragment était considéré comme tel. Pour 4/8 réponses on considérait le logiciel comme incapable d'identifier le fragment et une série de dépôts supplémentaire était requise. Enfin, pour chaque diagnostic posé, la classification issue de cette analyse des réponses données par le logiciel Biotyper® a été confrontée au diagnostic histologique définitif permettant de considérer la classification comme vrai positif (VP) vrai négatif (VN) faux positif (FP) ou faux négatif (FN) pour le calcul des sensibilités (Se) et spécificités (Sp), valeurs prédictives positives et négatives et rendement diagnostic selon les formules habituelles :

$$Se=VP/(VP+FN),$$

$$Sp=VN/(VN+FP), VPP=VP/(VP+FP), VPN=VN/(VN+FN), Rendement=(VP+VN)/(VP+VN+FP+FN).$$

Le tableau B ci-après indique les résultats des performances diagnostiques pour le diagnostic de cancer sur une cohorte de 196 échantillons de poumons testés en aveugle avec le logiciel Biotyper® :

Tableau B

|  | Tissu cancéreux | Tissu sain |
|---|---|---|
| Sensibilité : | 93.6% | 98.9% |
| Spécificité : | 70% | 92.9% |

Comme l'indique la spécificité basse de ce test lorsqu'une tumeur non cancéreuse est analysée, il existe un risque de sur-diagnostiquer un cancer. Par ailleurs, les méthodes d'imagerie de tumeur à forte composante inflammatoire ne permettent pas de les distinguer aisément du cancer et d'autre part le diagnostic histologique extemporané des tumeurs cancéreuses nécrosées n'est pas performant et le diagnostic de cancer risque d'être sous-estimé. Une autre approche méthodologique a alors été décidée basée sur un traitement de spectres et calcul pouvant être mis en oeuvre à l'aide d'un nouveau logiciel que rapporté ci-après.

**Exemple 4 :** Analyse de spectres de broyats de poumons sains, tumoraux cancéreux et tumoraux inflammatoires par le nouvel algorithme selon la présente invention.

A) Même si les spectres des tissus de poumons sains représentent un profil reproductible (figure 1), afin d'augmenter la détection d'une information contenue dans le tissu tumoral par élimination de l'information portée par le tissu pulmonaire sous-jacent, les spectres sont comparés 2 à deux et soustraits selon une méthode mise en oeuvre par un logiciel développé par les inventeurs ci-après dénommé IHU, **le** premier est un tissu sain, l'autre le tissu à analyser du même patient.

1) Le principe du logiciel IHU est original en ce qu'il ne réalise pas une étude par comparaison des seuls pics mais prend en compte la totalité de la courbe des spectres. Le logiciel développé travaille à partir de 2 spectres de spectrométrie de masse et comprend les étapes suivantes :

- Les 2 spectres sont représentés selon un axe des abscisses des masses, les intensités I exprimées en unité arbitraire et sont représentées en sens inverse avec le spectre de référence (poumon sain) au-dessus de l'axe des abscisses et le spectre de l'échantillon à analyser (poumon tumoral) en dessous. Ainsi, les alignements de pics relatifs au même composant ou décalages d'alignement éventuels des pics des 2 spectres apparaissent plus clairement. Un décalage de pic peut résulter d'une erreur pour des pics relatifs à un même composant et qui auraient dû être soustraits si le décalage n'excède pas 100Da ou le décalage peut au contraire correspondre à 2 pics relatifs à des composants différents.

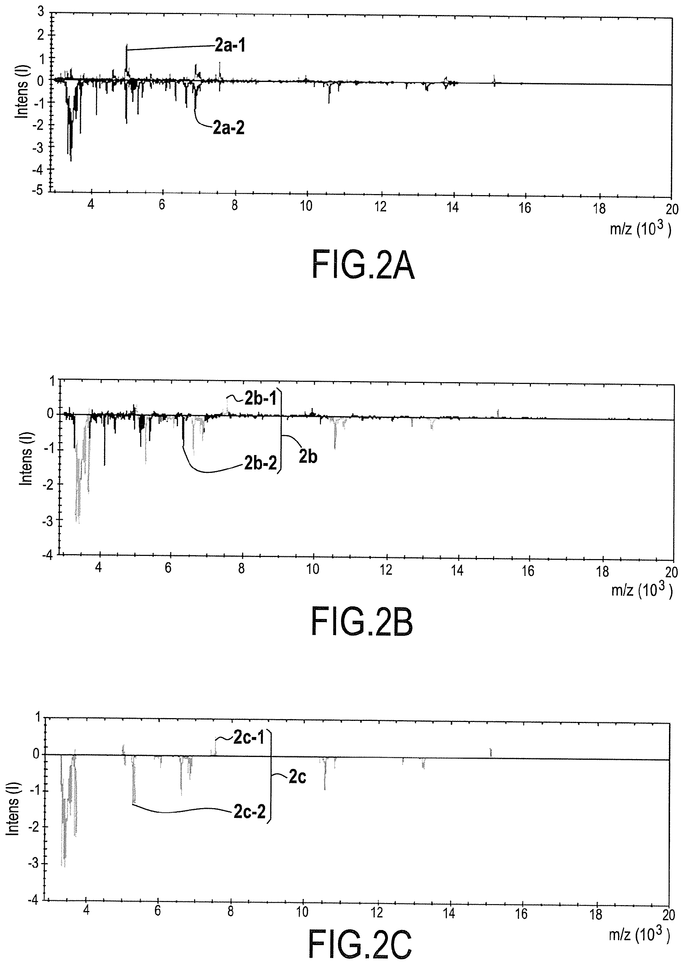

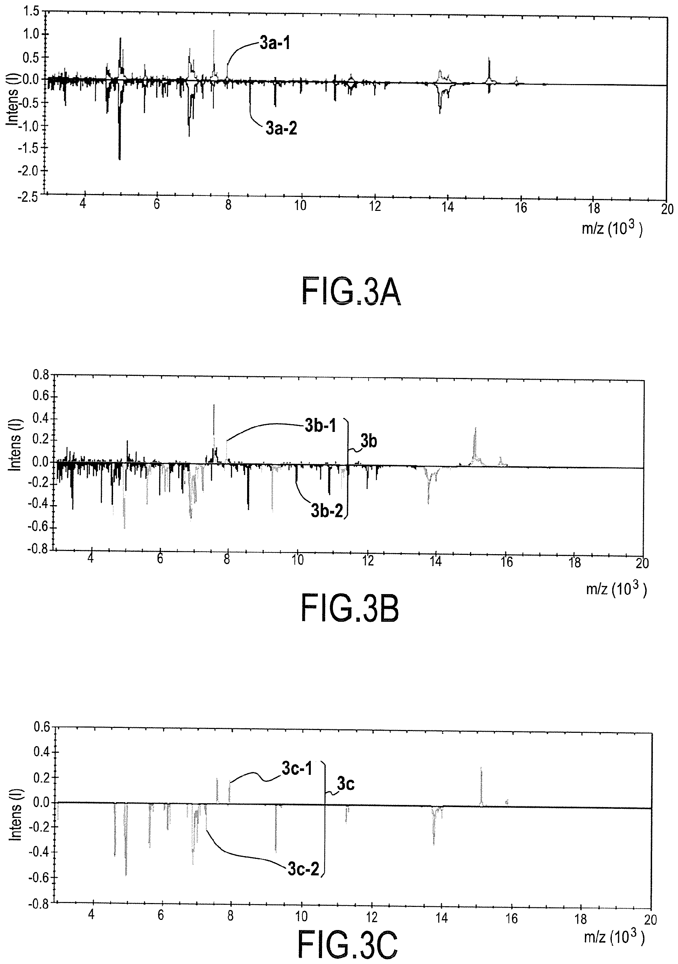

- Dans une première étape (figures 2A et 3A), les deux spectres spectre 1 (spectre de référence) et spectre 2 (spectre de l'échantillon à analyser) à comparer sont mis à l'échelle (en se basant sur l'intensité du pic de plus grande intensité), c'est-à-dire que pour chaque spectre les intensités y1 en ordonnée pour chaque valeur de x1 en abscisse (m/z) sont normalisées en divisant yi par l'intensité du pic de plus grande intensité du spectre. - Dans une deuxième étape (figures 2B et 3B), les deux spectres sont ensuite soustraits pour chaque valeur de xi, on calcule yi3=yi2-yi1 pour obtenir un spectre 3 = spectre 1-spectre 2, et

- dans une troisième étape (figure 2C et 3C),.le bruit de fond du spectre différence est enlevé en mettant à 0 les valeurs yi3 inférieur à un certain seuil, par exemple inférieur à Sy1=5% du pic de plus grande intensité tel que décrit ci-dessus en liaison avec l'étape i.3a, et on filtre par mise à 0 les valeurs de yi3 correspondant à des pics qui ne sont pas bien alignés entre les 2 spectres et n'ont pu être soustraits correctement tel que décrit ci-dessus en liaison avec l'étape i.3b avec une valeur Sy2 de 70% et Sx= (100Da), et

- à partir de ce spectre résultant après filtrage ci-dessus, on calcule un score différentiel normalisé qui est l'aire total résiduel du spectre (positive et négative) divisé par l'intensité total du premier spectre selon la formule $\sum_{i=0}^{i=n}|yi3|/\sum_{i=0}^{i=n}yi1$.

A titre illustratif, les figures 2A-2C et 3A-3C représentent les 2 spectres analysés et comparés d'échantillons de poumons sain (spectre de référence) et poumons à tumeurs non cancéreuses inflammatoire (Figures 2A-2B) et poumons à tumeurs non cancéreuses cancer (Figures 3A-3C) ou tumeur non cancéreuse non inflammatoire (non représentés) avec le nouveau logiciel IHU.

Sur la figure 2A sont représentés le spectre brut 2a-1 de broyat de tissu de poumon sain placé du côté positif de l'axe des ordonnées et le spectre brut 2a-2 de broyat de tissu de poumon tumoral inflammatoire non cancéreux placé du côté négatif. A ce stade, pour le spectre 2a-1 la somme $\sum_{i=0}^{i=n}yi1 = 541.45$ et, pour le spectre 2a-2 la somme $\sum_{i=0}^{i=n}yi2=1979.631$.

Sur la figure 2B sont représentés le spectre de soustraction 2b après normalisation et avant filtrage des spectres. Les pics résiduels du tissu de poumon sain 2b-1 étant portés en positif et les pics résiduels du tissu inflammatoire 2b-2 étant portés en négatif. A ce stade, pour le spectre 2b-1 la somme $\sum_{i=0}^{i=n}yi1$ $38,429$ et pour le spectre 2b-2 la somme $\sum_{i=0}^{i=n}yi2 = 1162,093$.

La figure 2C représente le spectre soustrait 2c après élimination du bruit de fond et filtrage puis le filtrage des pics mal soustraits du fait d'un décalage du spectre 2b de la figure 2B. Sur la figure 2C, il reste des pics résiduels du poumon sain 2c-1 en positif et des pics résiduels du poumon inflammatoire 2c-2 en négatif. A ce stade, pour le spectre 2c-1 la somme $\sum_{i=0}^{i=n}yi1 = 20.988$ et pour le spectre 2c-2 la somme $\sum_{i=0}^{i=n}yi2 = 1008.983$ et le score différentiel normalisé est de $\sum_{i=0}^{i=n}|yi3|/\sum_{i=0}^{i=n}yi1=1.902$.

La figue 3A représente les spectres bruts de broyats de tissu de poumon sain en 3a-1 et de tissu cancéreux 3a-2. A ce stade, pour le spectre 1 (3a-1) la somme $\sum_{i=0}^{i=n}yi1 = 524.587$ et, pour le spectre 2 (3a-2) la somme $\sum_{i=0}^{i=n}yi2=879.37$.

La figure 3B représente le spectre de soustraction 3b des deux spectres de la figure 3A après normalisation et avant filtrage. A ce stade, pour le spectre 3b-1 la somme $\sum_{i=0}^{i=n}yi1 = 36,379$ et pour le spectre 3b-2 la somme $\sum_{i=0}^{i=n}yi2 = 228,689$.

La figure 3C représente le spectre soustrait 3c après élimination du bruit de fond. A ce stade, pour le spectre 1 (3c-1) la somme $\sum_{i=0}^{i=n}yi1$ =14.643 et pour le spectre 2 (3c-2) la somme $\sum_{i=0}^{i=n}yi2 = 128.421$ et le score différentiel normalisé ou score IHU est de $\sum_{i=0}^{i=n}|yi3|/\sum_{i=0}^{i=n}yi1=0.273$.

Sur les figures 3B et 3C, les pics résiduels en positifs 3b-1 et 3c-1 proviennent du poumon sain et les pics résiduels 3b-2 et 3c-2 en négatifs proviennent du poumon cancéreux.

Sur les figures 2B et 3B, on voit pour certaines valeurs de xi et xi' avec xi'-xi inférieur à 10Da, il ya des changements de signes des intensités résultantes yi3=yi2-yi1 et yi3'=yi2'-yi1'.Sur les figures2C et 3C, il n'y a plus de valeurs de xi avec des changements de signes des intensités résultantes entre xi et xi' pour xi'-xi inférieur à 100Da.Les pics de couleur gris plus foncée des figures 2B et 3B sont les pics qui sont éliminés et filtrés sur les figures 2C et 3C.

Les spectres bruts de broyat de tissus de poumon sain et respectivement de tissu de poumon tumoral non cancéreux non inflammatoire ont été réalisé pareillement (non représentés). Le score IHU brut calculé est de 0,012.

B) Des analyses ci-dessus faites pour un tissu à tumeur inflammatoire (figures 2A à 2C), pour un tissu à tumeur cancéreuse (figure 3A à 3C) et pour un tissu avec une tumeur bénigne (non représenté), on peut voir que la soustraction de chaque nature de tissu (tumeur non cancéreuse bénigne inflammatoire, tumeur cancéreuse, tumeur non cancéreuse bénigne non inflammatoire) avec le tissus sain donne un spectre résiduel diffèrent et caractéristique. Les spectres résiduels correspondent à des pics soit présent dans un fragment et absent dans l'autre, soit à une intensité suffisamment différente entre les deux tissus. En utilisant le logiciel IHU qui permet de calculer l'intensité résiduelle de deux spectres soustraits comme décrit ci-dessus, on a pu montrer qu'il était possible de discriminer chaque type de tissus en fonction de la valeur absolue du score. En particulier la discrimination entre les tissus tumoraux cancéreux et tissus tumoraux non cancéreux inflammatoires qui sont difficiles à discriminer avec le logiciel Biotyper®.

C) Les différents essais sur une cohorte de 196 échantillons analysés 2 à 2 entre le fragment sain et le fragment tumoral d'un même patient en fonction de la nature de la tumeur, permettent de conclure que pour ce type d'échantillons de poumons avec tumeur, avec le paramétrage des intensités et différentes valeurs seuils décrit ci-dessus, l'on détermine l'identité de nature ou de qualité de l'échantillon à analyser et de l'échantillon de référence comme suit :

- si le score différentiel entre l'échantillon à analyser et un échantillon de référence de même nature sain est inférieur à une valeur inférieure à 0,0035, on conclut à l'identité des deux échantillons, et on conclut à une absence de tumeur (poumon sain),

- si le score différentiel est de 0,005 à 0,05, on conclut à une tumeur non inflammatoire, celle-ci pouvant être soit un cancer soit une tumeur non cancéreuse non inflammatoire - si le score différentiel entre l'échantillon à analyser et un échantillon de référence de même nature sain est entre 0,05 et 0,5, on conclut à la présence d'une tumeur cancéreuse et l'absence de tumeur non cancéreuse inflammatoire,

- si le score différentiel entre l'échantillon à analyser et un échantillon de référence de même nature sain est supérieur à 0,5, on conclut à la présence d'une tumeur non cancéreuse inflammatoire et l'absence de cancer.

Le score IHU seul ne permet pas de discriminer nettement le cancer des tumeurs non cancéreuses non inflammatoires de manière certaine (voir paragraphe D ci-après). Une stratégie combinant les résultats fournis par le logiciel Biotyper® aux informations du score différentiel donné par le logiciel IHU tel que ci-après exposée au paragraphe D) doit alors être appliquée.

Le tableau C ci-après présente les scores différentiels ou scores IHU moyens des différents tissus obtenu sur une cohorte de 196 échantillons soustraits 2 à 2 entre le fragment sain et le fragment tumoral d'un même patient en fonction de la nature de la tumeur.

Tableau C :

|  | moyenne | Ecart type |
|---|---|---|
| Non cancéreux inflammatoires | 1,96 | 2,9 |
| cancéreux | 0,23 | 0,152 |
| non cancéreux non inflammatoire | 0,07 | 0,055 |

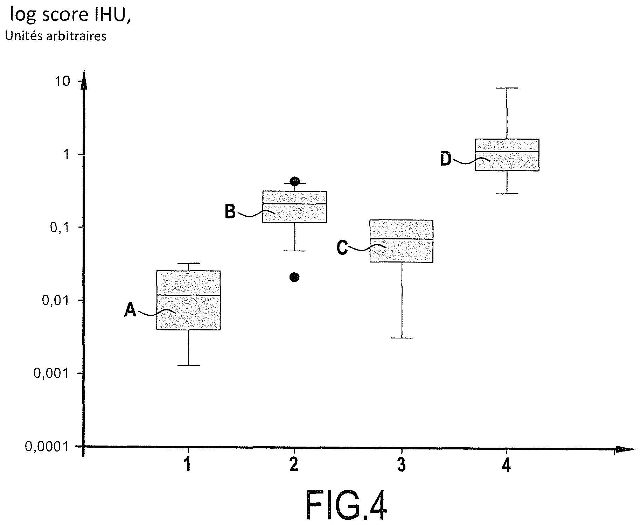

Les inventeurs ont vérifié la reproductibilité et la spécificité des spectres des différents types de poumons (figure 4) par une représentation bidimensionnelle. Ils ont constaté que les signatures en spectrométrie de masse MALDI-TOF, avec le logiciel IHU sont remarquablement homogènes au sein de chaque type de poumon, comme le montre la figure 4.Sur la figure 4, la dispersion au sein d'une cohorte de 196 échantillons, du score calculé avec le logiciel IHU pour différentes sous-classes d'échantillons de poumons sains (A), cancéreux (B), tumeurs bénignes non inflammatoires (C),tumeur bénigne inflammatoires (D) sont représentés par la représentation habituelle pour l'homme de l'art dite des « boites à moustaches » ou « Box Plots ». Les limites des boites représentent les $25^{ième}$-$75^{ième}$ percentiles (c'est-à-dire la valeur du score telle que 25% des fragments du sous-groupe ont obtenu une valeur plus basse pour le 25$^{\text{ième}}$ -et 75% des fragments du sous-groupe ont obtenu une valeur plus élevée pour le 75$^{\text{ième}}$) L'intervalle compris entre le 25$^{\text{ième}}$-75$^{\text{ième}}$ percentiles correspond donc à l'intervalle à l'intérieur duquel sont situés les 50% des données centrales ici représenté par la hauteur de la boite.. Les lignes verticales représentent les valeurs extrêmes. La symétrie des boites par rapport à la médiane (ligne horizontale) et la faible hauteur des boites témoignent du faible écart type illustrant la faible dispersion de la variable. L'absence de recouvrement entre les groupes de poumons sains, cancéreux et tumeurs non cancéreuses (bénignes) à forte composante inflammatoire et des tumeurs non cancéreuses (bénignes) non inflammatoires témoigne de la bonne discrimination de ces classes par les scores IHU. Cette bonne discrimination est attestée par une différence significative telle que calculée statistiquement en utilisant un test non paramétrique de Kruskall-Wallis et dont le degré de significativité entre les groupes est ici de 0,001.

D) En utilisant le logiciel Biotyper® combiné aux informations du score différentiel donné par le logiciel IHU, la prédiction et la sensibilité du diagnostic ont été améliorées. En effet, avec le logiciel IHU pour 96 échantillons de tumeurs (76 cancers et 20 Tumeurs Non Cancéreuses), et en ciblant une valeur du score entre 0,5 et 0,05, la sensibilité (Se) et la spécificité (Sp) du score IHU pour le diagnostic de cancer était de 88 % et 65% respectivement. Ces valeurs de sensibilité et spécificité pour le diagnostic de cancer avec le score IHU sont améliorées et supérieures à celles du logiciel Biotyper si on retire les tumeurs bénignes non inflammatoires portant à 87 l'effectif (Se=97,3%. et Sp=81,1%, rendement = 91% avec le scope IHU).

Il apparaît toutefois que le diagnostic de cancer manque de spécificité avec la classification Biotyper seule (70%) comme avec la classification par le logiciel IHU si on inclut tous les types de tumeurs. Cela qui signifie qu'un fragment tumoral de nature non cancéreuse risque d'être classé cancer par excès avec ces deux méthodes.

Compte tenu de la bonne sensibilité du logiciel Biotyper® pour diagnostiquer les lésions cancéreuses mais de sa moins bonne spécificité pour éliminer les lésions Tumorales non cancéreuses, lorsqu'un fragment tumoral suspect est classé cancéreux d'après le logiciel Biotyper®, le score IHU a été calculé. Un score IHU< 0,5 permettait d'affecter ce fragment à la classe cancer. Un score ≥ 0,5 permettait d'affecter l'échantillon à la classe des tumeurs non cancéreuses.

Plus précisément, devant un tissu pulmonaire suspect, les spectres correspondants sont analysés avec le logiciel Biotyper® en mettant en oeuvre la base de données complète de spectres de poumons humains. Si la réponse est : 'Non Cancer' avec plus de 5 réponses certaines, on considère que le tissu est bénin et on ne réalise pas de résection chirurgicale étendue.

- Si la réponse de Biotyper® est 'Cancer', on calcule le score IHU, s'il est < 0,5 on retient le diagnostic de Cancer, s'il est ≥ 0,5 on ne retient pas le diagnostic de cancer.
- Si la réponse est 'Cancer' avec moins de 5 réponses certaines, on ne retient pas le diagnostic de cancer.
- Ainsi, lorsque le diagnostic de cancer n'est pas retenu, le tissu est considéré bénin et on ne réalise pas de résection chirurgicale étendue.

Cette stratégie diagnostique combinée tel que décrit ci-dessus permet de classer correctement les échantillons cancéreux avec une Sensibilité de 91% et une Spécificité de 90%.

L'avantage du score IHU est de pouvoir discriminer de manière plus fiable les tumeurs cancéreuses et tumeurs inflammatoires.

**Exemple 5:** Protocole de traitement et préparation d'un extrait protéique d'un concentré plaquettaire.

A) On a tenté de réaliser un spectre de concentré plaquettaire brut en suivant le protocole conventionnel suivant sans succès.

Sur une plaque d'un appareil spectromètre Microflex LT de Bruker Daltonics™, on dépose 1 µl de concentré plaquettaire puis 1 µl de solution de matrice HCCA décrite ci-après et on laisse sécher à température ambiante.

Le concentré plaquettaire était obtenu à partir d'une poche d'échantillonnage de CP stérile apyrogène de 30 ml de la société MacoPharma REF: VSE 0000A.

Le spectre est acquis en mode automatique sur un Microflex LT en utilisant la méthode suivante décrite ci-après. Aucun spectre exploitable n'a pu être obtenu par le logiciel Biotyper® ou le nouveau logiciel décrit ci-après

a.1. La préparation de la solution de matrice (HCCA saturée) comprend les étapes suivantes consistant à :.

- Introduire dans un tube polypropylène de 1.5 ml, 2 pointes de spatules d'acide alpha-cyano-4-hydroxycinnamique (HCCA),

- Ajouter 500 µl d'acétonitrile , 475 µl d'eau HPLC et 25 µl de TFA (acide trifluoroacetique),

- Mélanger au Vortex ou secouer vigoureusement, Soniquer 10 min puis centrifuger 5 min à 13000 g,

- Transférer le surnageant dans un tube polypropylène 1.5 ml propre.

a.2. La méthode d'acquisition des spectres comprend les étapes et caractéristiques suivantes :

- introduire la plaque (cible Microflex modèle de référence 224989 dénommé MSP 96) contenant les dépôts dans le spectromètre de masse Microflex LT,

- Démarrer l'ordinateur associé au spectromètre pour lancer les analyses, et

- Lancer l'acquisition des spectres de masse MALDI-TOF.

Les paramètres appliqués au spectromètre de masse sont les suivants :

- Mode linéaire positif, Electrode IS1 : 20,00 kV, Electrode IS2 : 18,05 kV, Electrode de focalisation : 6 kV, Fréquence du laser : 60Hz, Gain du détecteur : 8,8 X, Post-ion-extraction (PIE) : 120 ns, Gamme de masse : de 700 à 20000 Da.

Les spectres sont obtenus via une méthode automatique contrôlée par le logiciel FLEXCONTROL® du fabricant BRUKER DALTONICS® les paramètres sont les suivants pour chaque spot :

- Nombres séries de 100tirs: 20 sur des positions différentes du spot (20X1000 = 2000 spectres)

- Afin que les tirs aient balayé l'ensemble de l'échantillon de façon homogène l'acquisition se fait sur 6 positions différentes selon une géométrie hexagonale.

- Pré-tirs : 10 tirs à 40% de la puissance maximum laser servent à dessaler l'échantillon (contamination par les sels minéraux)

- Puissance laser : 30 % à 45 % régulée via le logiciel FLEXCONTROL®

- Sélection du spectre : sur une gamme de masse de 4 000 à 10 000 Da.

L'acquisition des spectres est au final réalisée via le logiciel Biotyper® du fabricant BRUKER DALTONICS® qui permet d'appliquer la méthode automatique décrite ci-dessus à une série d'échantillons en prenant en compte les critères mentionnés précédemment et un score de qualité ou score de validation qui représentent l'addition de 2 notes n1 + n2.

B) Après de multiples essais avec différents réactifs et traitements divers, le protocole suivant a permis d'obtenir des spectres de spectrométrie de masse maldi-tof selon la méthode ci-dessus décrite.

Le protocole de traitement et préparation d'un extrait protéique de lysat cellulaire de CP comprend les étapes successive suivantes consistant à :

- mettre 1 ml de concentré plaquettaire dans un tube eppendorf, et le centrifuger à 14000G pendant 5 min. puis éliminer le surnageant, et

- ajouter 900 µl d'eau et 300 µl de solution de saponine (0.5g/ml dans de l'eau) dans le culot de centrifugation, puis mélanger dans un Vortex, puis centrifuger à 14000G pendant 5 min, puis éliminer le surnagent, et

- laver le culot avec 1 ml d'eau, après avoir centrifugé 5 min à 14000G, éliminer le surnageant, puis

- ajouter 25 µl d'une solution d'acide formique à 70% ( 700microlitre d'acide formique dans 300 microlitre d'eau) dans le culot de centrifugation , puis mélanger par mouvement aller et retour de bas en haut avec une micropipette Eppendorf , puis rajouter encore 25 μl d'acétonitrile puret mélanger par mouvement de up et down avec la micropipette, puis centrifuger à 14000G pendant 5 min, puis

- récupérer la solution claire de surnageant correspondant à un d'extrait protéique de lysat cellulaire de l'échantillon de CP d'origine.

La centrifugeuse était une centrifugeuse de la société THERMOSCIENTIFIC (USA) de référence HERAEUS®-Biofuge pico.

Des spectres ont été obtenus comme montré à la figure 1 après dépose de 1 μl de la solution claire sur le cible MALDI-TOF (MSP 96, Bruker Daltonics référence 224989), puis séchage à température ambiante, puis dépose de 1 μl de solution de matrice et à nouveau séchage à température ambiante. Le spectre est acquis en mode automatique sur un Microflex® LT en utilisant la méthode décrite précédemment.

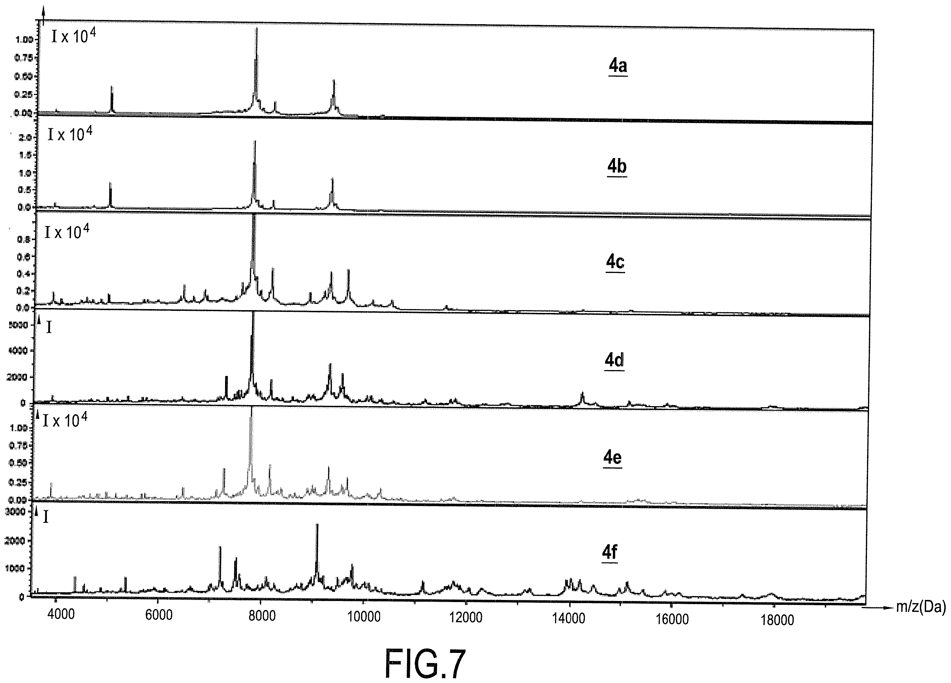

Les spectres 4a et 4b de la figure 7présentent la liste des pics rapportés dans le tableau A mentionné ci-dessus.

Les échantillons de CP ont été traités par l'étape d'extraction selon la méthode décrite ci-dessus. Les spectres ont été obtenus sur des extraits de 35 poches de CP d'individus de groupes sanguins variables. On observe une reproductibilité des spectres inter-poches (n=35) de concentrés de plaquettes non infectées quelques soit le groupe sanguin.

Les concentrés de plaquettes ont été conservés pendant 9 jours. Chaque jour, les échantillons ont été traités par l'étape d'extraction Acide formique/Acétonitrile selon la méthode décrite ci-dessus. Les spectres d'extrait protéique de CP obtenus le premier jour (J1) et le neuvième jour (J9) ont été comparés et on observe une reproductibilité des spectres intra-poches au cours du temps. Le spectre des plaquettes à J9 est sensiblement identique au spectre des plaquettes à J1.

**Exemple 6:** Préparation d'extrait protéique d'échantillon de concentrés plaquettaires contaminés par des bactéries.

1) Préparation des inocula bactériens :

On a réalisé le protocole comprenant les étapes successives suivantes consistant à :

- mettre en culture le germe d'intérêt sur une gélose au sang, puis

- après 24 heures de culture, prélever l'ensemble des colonies afin de préparer une solution d'inoculum dans 15 ml d'eau stérile, puis

- verser un aliquote de1 ml dans 15 tubes eppendorfs, les diluer au 10eme en cascade, puis

- déposer 100 μl des différentes dilutions sur une gélose au sang et étaler de façon homogène à l'aide d'un râteau d'étalement, puis

- laisser incuber 24H à 37°C, puis compter les colonies apparues sur une dilution donnant de 100 à 200 colonies.

La concentration de l'inoculum obtenu en UFC /ml est le nombre de colonies X (1/la dilution) X10UFC : unité formant colonies.

2) Préparation de concentré plaquettaire contaminé.
On a réalisé le protocole comprenant les étapes successives suivantes consistant à :

- prélever 900 μl de concentré plaquettaire à partir de la poche et les mettre dans un tube eppendorf de 2 ml, puis ajouter 100 μl de solution bactérienne ci-dessus à des concentrations de 102 à 108 ufc/ml (soit en concentration finale de 101 à 107 ufc/ml), puis

- incuber les tubes de 0 à 24 heures à 37°C.

Ensuite on réalise le même traitement et préparation d'extrait protéique que décrit ci-dessus consistant à :

- à chaque temps, prendre le tube eppendorf de 2ml, le centrifuger à 14000G pendant 5 min et éliminer le surnageant, et

- ajouter 900 µl d'eau et 300 µl de solution de saponine, vortexer puis centrifuger à 14000G pendant 5 min et éliminer le surnageant, et

- laver avec 1 ml d'eau, après avoir centrifugé 5 min à 14000G, éliminer le surnageant, puis

- ajouter 25 µl d'acide formique à 70%, mélanger par mouvement avec la micropipette, puis rajouter 25 µl d'acétonitrile et mélanger par mouvement avec la micropipette, puis centrifuger à 14000G pendant 5 min, puis

- déposer 1 µl de la solution claire de surnageant sur la cible MALDI-TOF, laisser sécher à température ambiante. Déposer 1 µl de solution de matrice et laisser sécher à température ambiante. Le spectre est acquis en mode automatique sur un Microflex LT en utilisant la méthode décrite précédemment à l'exemple 1.

3) Protocole d'incubation avec un milieu de culture des concentrés de plaquettes contaminés.
On a réalisé le protocole comprenant les étapes successives suivantes consistant à :

- prélever 900 µl de concentré plaquettaire à partir de la poche et les mettre dans un tube eppendorf de 2ml, puis ajouter 100 µl de solution bactérienne à des concentrations de 102 à 108 (soit en concentration finale de 101 à 107), puis

- ajouter 1 ml de milieu de culture dans chaque tube, et

- incuber de 0 à 24 heures à 37°C.

Des essais ont été réalisés avec plusieurs milieux de cultures dont le milieu trypticase Soja de composition suivante par litre d'eau purifiée :

- digestion pancréatique de caséine :............... 17,0 g

- digestion papaïque de semoule de soja ............. 3,0 g

- NaCl : :........................................................... 5,0 g

- K2PO4 :............................................................ 2,5 g

- Dextrose : ....................................................... 2,5 g

Ensuite, on a réalisé le même traitement et préparation d'extrait protéique et même protocole d'acquisition de spectre par spectrométrie de masse maldi-tof avec le même appareillage Microflex que décrit ci-dessus.
4) Des poches de concentrés plaquettaires contaminés ont été réalisées selon le protocole comprenant les étapes successives suivantes consistant à :

- prélever 2 fois 1 ml de concentré plaquettaire CP (qui serviront de témoins négatifs), puis

- ensemencer d'une dilution bactérienne de 1 ml à une concentration prédéfinie dans de l'eau dans un tube eppendorf de 2 ml, par injection avec une seringue dans une tubulure du MCP au plus près de l'entrée de la poche, puis

- réaliser une soudure de la tubulure avec une soudeuse automatique en amont du point d'injection, puis

- remuer le CP pour obtenir une répartition homogène des germes.

L'échantillonnage est réalisé à différents temps via l'utilisation d'une connexion de la tubulure du CP. Une fois le volume désiré récupéré dans une poche d'échantillonnage et une soudure de la tubulure du CP est réalisée.

Le suivi de la contamination des poches de concentré plaquettaires comprenant les étapes successives suivantes consistant à :

- après la contamination, prélever 3 échantillons de volumes 1ml, 8ml et 16ml et appliquer le protocole d'incubation dans les plaquettes+milieu de culture décrit paragraphe 3) ci-dessus, puis

- après 24 heures incubation à 24°C sous agitation (protocole standard de conservation des concentrés de plaquettes) prélever 3 échantillons de volumes 1ml, 8ml et 16ml et appliquer le protocole d'incubation dans les plaquettes+milieu de culture décrit paragraphe 3) ci-dessus.

La comparaison de 2 spectres a été réalisée par le logiciel Biotyper3.0 et par un nouveau logiciel exécutant nouvel algorithme fourni par la présente invention comme décrit à l'exemple 7 ci-après.

**Exemple 7 :** Analyse des spectres de masse pour la détection de contamination bactérienne.

A) Analyse par le logiciel Biotyper®.

La méthode a été décrite ci-dessus à l'exemple 3-A).

En résumé, le score d'identification est pour une bactérie, selon les valeurs suivantes:

- de 0 à 1,69 : identification non fiable

- de 1,7 à 1,99.0 : identification possible, notamment du genre pour une bactérie

- de 2.0 à 3.0 : identification certaine, notamment du genre et de l'espèce pour une bactérie.

B) Nouveau logiciel dénommé logiciel IHU exécutant un nouvel algorithme de comparaison de 2 spectres selon la présente invention.

Le principe du logiciel a été décrit ci-dessus à l'exemple 4.

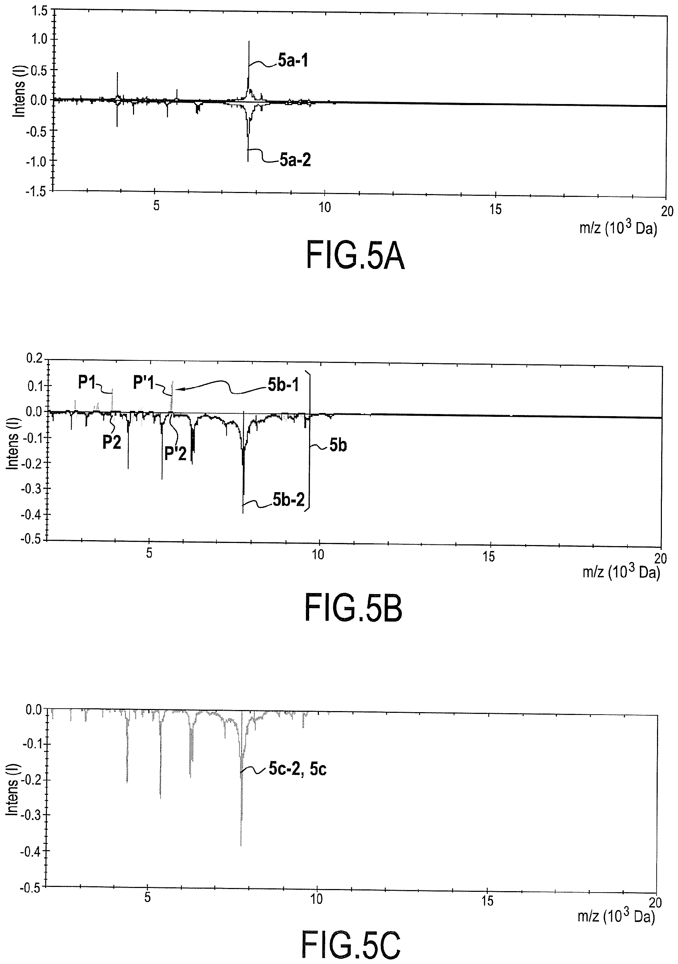

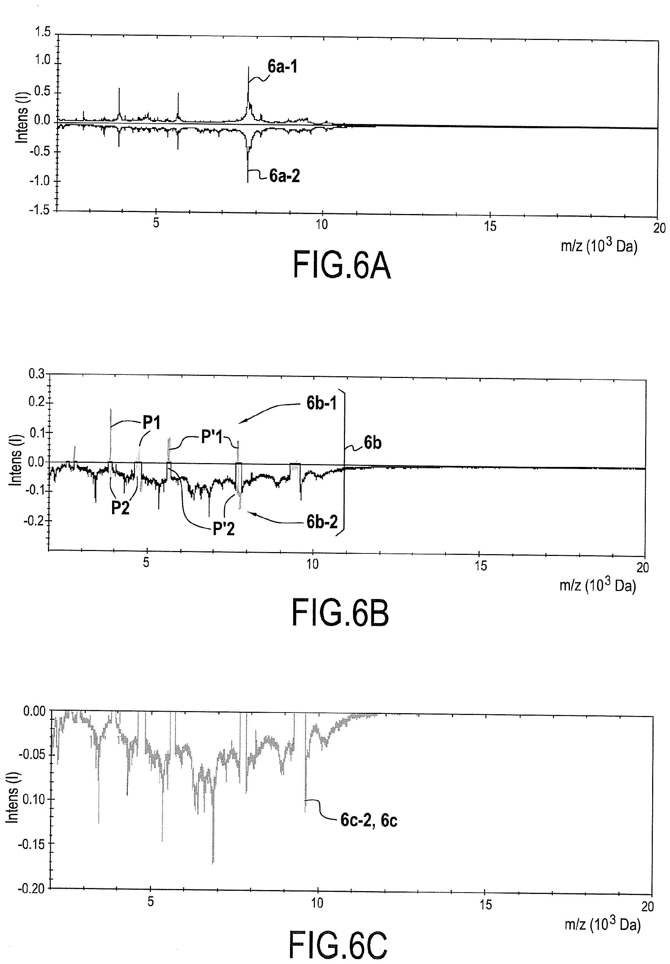

A titre illustratif, les figures 5A à 5C et 6A à 6C représentent les spectres des échantillons suivants analysés et comparés avec le nouveau logiciel IHU.

Les figures 5A et 6A montrent les spectres bruts obtenus pour des extraits protéiques obtenus à partir d'échantillons de concentré de plaquettes contaminées par $10^7$ *E. coli* par ml (5a-2, figure 5A) et par $10^7$ *S.aureus* par ml (6a-1 figure 6A), ainsi que les spectres des mêmes concentrés plaquettaires non contaminés (5a-1, figure 5A et 6a-1 figure 6A).

Sur les figures 5B et 6B, on a représenté les spectres 5b et 6b de soustraction des deux spectres 1 (5a-1 / 6a-1) et 2 (5a-2 / 6a-2) après leur normalisation. Sur les figures 5C et 6C, on a représenté les spectres 5c et +6c de soustraction après élimination du bruit de fond et après le filtrage des pics mal soustraits des spectres 5b et 6b des figures 5B et 6B.

Sur les figures 5B-5C et 6B-6C, des pics résiduels en positifs 5b-1 et respectivement 6b-1, proviennent concentré de plaquettes non contaminé et les pics résiduels en négatifs 5b-2, 5c-2 et respectivement 6b-2,6c-2 proviennent de concentré de plaquettes contaminé. Sur les figures 5C et 6C, il n'y a plus de pics résiduels en positifs.

Sur les figures 5B et 6B, on voit pour certaines valeurs de xi et xi' avec xi'-xi inférieur à 100Da, il y a des paires de pics alignées p1/p2 et p1'/p2' avec changements de signes des intensités résultantes yi3=yi2-yi1 et yi3'=yi2'-yi1'.Sur les figures 5C et 6C, il n'y a plus de valeurs de xi avec des changements de signes des intensités résultantes entre xi et xi' pour xi'-xi inférieur à 100Da .

Les pics de couleur gris plus clair des figures 5B et 6B sont les pics qui sont éliminés et filtrés sur les figures 5C et 6C.

Sur les figure 5A et 6A, pour le spectre 1 (5a-1 et 6a-1) la somme $\sum_{i=0}^{i=n} yi1 = 247.741$ (figure 5A) et 623,235 (figure 6A), pour le spectre 2 (5a-2 et 6a-2) la somme $\sum_{i=0}^{i=n} yi2 = 495,99$ (figure 5A) et 14231,294 (figure 6A).

Figures 5B et 6B, pour le spectre 1 (5b-1 et 6b-1) la somme $\sum_{i=0}^{i=n} yi1 = 0.006$ (figure 5B) et 0 (figure 6B), et pour le spectre 2 (5b-2 et 6b-2) la somme $\sum_{i=0}^{i=n} yi2 = 222.533$ (figure 5B) et 579,794 (figure 6B), et le score différentiel normalisé avant filtrage est de $\sum_{i=0}^{i=n} |yi3| \, / \sum_{i=0}^{i=n} yi1 = 0,898$ (figure 5B) et 0,917(figure 6B).

Sur les figures 5C et 6C, pour les spectres 1 (5c-1 et 6c-1) la somme $\sum_{i=0}^{i=n} yi1 = 0$, et pour les spectres 2 la somme $\sum_{i=0}^{i=n} yi2 = 137.901$ (figure 5C) et430, 243 (figure 6C),et le score différentiel normalisé après filtrage (score IHU définitif) est de $\sum_{i=0}^{i=n} |yi3| \, / \sum_{i=0}^{i=n} yi1 = 0.557$ (figure 5C) et 0,681 (figure 6C).

Les différents essais permettent de conclure que pour ce type d'échantillons de concentrés de plaquettes contaminés par des bactéries, avec les paramétrages des différentes valeurs seuils mentionnés ci-dessus, l'on détermine ici la présence ou l'absence d'une contamination de l'échantillon si le score différentiel normalisé calculé sur un échantillon contaminé à t=0par rapport à un échantillon de référence non contaminé comme suit:

- si le score différentiel est inférieur à une valeur v1, v1 étant de par exemple notamment égale à 0.2, l'identité des deux échantillons est certaine, et donc l'absence de contamination est certaine

- si le score différentiel est compris entre v1 et v2, v2 étant par exemple égale à 0.5, l'identité des deux échantillons est possible, et donc la présence de contamination est possible, et

- si le score différentiel est supérieur à v2, il n'y a pas d'identité des deux échantillons et donc la présence de contamination est certaine.

Plus généralement, les différents essais permettent de conclure qu'on peut paramétrer la méthode pour déterminer la présence d'une contamination de l'échantillon si le score différentiel normalisé calculé sur un échantillon incubé pendant un temps t entre 0 et 24H par rapport à un échantillon de référence contaminé par la même bactérie à t=0 ou par rapport à un échantillon de même nature non contaminé dépasse un seuil N1, N1 étant de 0.1 à 10.

**Exemple 8:** Résultats d'analyse des spectres de masse pour la détection de contamination bactérienne dans des échantillons de concentrés plaquettes contaminés (CP) avant incubation et sans milieu de culture.

1) On a réalisé des identifications bactériennes après inoculation de fortes concentrations de solutions bactériennes, un inoculum de solutions bactériennes de concentration supérieur à $10^7$ ufc/ml de différentes bactéries ayant été ajouté respectivement à différents aux échantillons de CP non contaminés.

Les échantillons contaminés ont subi une méthode de traitement et d'extraction décrite au paragraphe B) de l'exemple 5 ci-dessus pour analyse spectrale au spectromètre de masse maldi-tof décrit ci-dessus. Les échantillons n'ont pas été incubés à 37°C et ne comportaient pas de milieu de culture

Les spectres obtenus ont tout d'abord été analysés par le logiciel Biotyper®. Cette analyse a eu lieu sans incubation, immédiatement après l'inoculation.

La figure 7 présente des exemples de spectres obtenus à partir d'échantillon non infectés c'est-à-dire avant inoculation (spectres 4a et 4b), et spectres obtenus à partir d'échantillon après inoculation par *Staphylococcus aureus* (spectre 4c), *Escherichia coli* (spectre 4d), *Providencia stuartii* (spectre 4e) et *Pseudomonas aeruginosa* (spectre 4f) et pour de fortes concentrations (supérieures à $10^7$ ufc/mL).

Les pics spécifiques de CP non contaminés n'occultent pas la mise en évidence de pics bactériens. Les flèches montrent la présence de pics supplémentaires dans les spectres 4c à 4f provenant d'échantillons de CP infectés qui correspondent à la présence de protéines bactériennes supplémentaires. La présence de ces pics supplémentaires traduit l'état de contamination. Par ailleurs, on observe comme attendu des pics différents en fonction des espèces.

Les spectres ont été analysés par le logiciel Biotyper®. Chaque expérience a été réalisée en quadruple.

Un score d'identification selon le logiciel Biotyper® supérieur à 2 est obtenu qui permet une identification du genre et de l'espèce de chaque bactérie par rapport aux spectres de références des dites bactéries de la base de données enregistrée dans Biotyper, c'est-à-dire des spectres de références réalisés sur des bactéries entières et non pas des échantillons de CP infectés et traités selon la présente invention. Les valeurs d'indice ou score d'identification S1 obtenues avec le logiciel BIOTYPER® pour les spectres obtenus à partir d'échantillons de CP infectés par les différentes bactéries au cours de différents essais sont globalement satisfaisants (supérieur à 2) pour de fortes concentrations bactériennes ($10^7$/ml, sans incubation).

2) Toutefois, comme décrit aux exemples 9 et 10 ci-après, pour des concentrations inférieures et/ou pour certaines bactéries, le score d'identification de BIOTYPER® n'est pas satisfaisant, car l'adjonction des pics spécifiques des composants provenant des plaquettes peut l'induire en erreur de sorte que le logiciel rend alors des résultats faussement négatifs (score trop bas) ou il se trompe dans l'identification proposée voire dans la détection de bactérie.

Ainsi, comme rapporté à l'exemple 9, pour Klebsiella Pneumoniae l'identification par Biotyper n'a pas été possible à partir d'extrait protéiques provenant d'échantillon de CP infectés par la dite bactérie quelques soit la concentration en bactérie et ce même après 16h d'incubation.

Il reste que la contamination des CP induit une modification des spectres de sorte que l'observation des spectres est suffisante lorsque l'on ne vise pas l'identification de la bactérie contaminante mais que l'on vise essentiellement la détection d'une contamination par une bactérie quelconque. Dans ce cas, la simple présence de pics additionnels par rapport au spectre caractéristiques de CP non infecté (tableau 1) est considérée comme anormal et conduit au retrait de la poche de CP analysée comme pouvant être contaminée. Il convient alors dans un second temps d'identifier si oui ou non il y a vraiment une bactérie mais cette démarche secondaire a un intérêt rétrospectif et épidémiologique, et peut se faire via des techniques classiques: culture sur gélose, analyse de spectre de masse MALDI-TOF classique après isolement de la bactérie.

C'est devant les insuffisances du logiciel Biotyper® qu'il a été décidé de développer et d'utiliser une nouvelle méthode de suivi de la pousse bactérienne résultant de l'incubation des échantillons infectés en présence milieu de culture et une nouvelle méthode d'analyse comparative des spectres tel que décrit à l'exemple 10 ci-après.

Cette nouvelle méthode d'analyse spectrale ne vise pas ici l'identification mais vise essentiellement la détection d'une contamination par une bactérie par suivi de l'évolution des spectres résultant de la pousse bactérienne dans l'échantillon testé décrit à l'exemple 9 ci-après.

**Exemple 9.** Identification bactérienne après inoculation de concentrations décroissantes de solutions bactériennes et analyse en cinétique du suivi de la pousse bactérienne sans ajout de milieu de culture.

Des solutions de concentrations décroissantes de bactéries ont été inoculées aux CP. L'analyse a été pratiquée sur le logiciel Biotyper® selon une cinétique allant de t=0 à t=24h.Les échantillons ont été préparés selon le protocole décrit à l'exemple 3 ci-dessus. Les résultats présentés ici ont été obtenus avec *E.coli, E.faecalis et K Pneumoniae.*

Les valeurs du score d'identification de Biotyper® de spectres ont été calculées à partir d'extraits protéiques d'échantillons de CP contenant des concentrations initiales croissantes d'Escherichia Coli de $10^2$ à $10^7$ ufc/ml sur une période de 24 heures.

Une identification avec un score supérieur à 2 de la bactérie a été obtenue par le logiciel BIOTYPER en 6 heures suite à une contamination du concentré plaquettaire par 1 ml d'une solution bactérienne de concentration $10^2$ ufc/mL. Dans ces conditions expérimentales, c'est-à-dire en absence d'ajout de milieu de culture, une identification d'espèce est donc obtenue pour E Coli après 6 heures d'incubation pour une concentration de $10^2$ ufc/ml.

Les spectres montrent que les pics spécifiques de CP non contaminés n'occultent pas la mise en évidence de pics bactériens de CP contaminés. Les pics bactériens supplémentaires dans le spectre provenant d'échantillons de CP infectés correspondent à la présence de protéines bactériennes supplémentaires. Des expériences similaires ont été réalisées avec différentes souches bactériennes.

Les résultats obtenus avec *Klebsiella Pneumoniae,* quelques soit la concentration initiale de l'échantillon de $10^2$ à $10^7$ ufc/ml , quelques soit le temps d'incubation de 0 à 16 heures à 37°C, étaient négatifs avec le logiciel Biotyper® il n'y a pas de variation significative des spectres représentant une prolifération bactérienne le score d'identification par Biotyper étant toujours inférieur à 1.2.Les scores d'identification obtenus par le logiciel Biotyper® ne permettent donc pas d'identifier la présence de la bactérie dans l'échantillon.

Des résultats négatifs similaires ont été obtenus avec d'autres souches bactériennes (résultats non présentés) à des concentrations inférieures à $10^7$ ufc/ml pour les espèces bactériennes de la liste de l'EFS (Etablissement public Français du Sang).

Au regard de ces résultats, les inventeurs ont décidé d'incuber les préparations de CP infectés dans un milieu de culture selon le protocole décrit ci-dessus et ont obtenu les résultats décrits aux exemples 10 et 11 ci-après.

**Exemple 10:** Analyse des CP après contamination bactérienne et ajout d'un milieu de culture et suivi de la pousse bactérienne avec le score d'identification du logiciel Biotyper.

Pour favoriser et accélérer la pousse bactérienne et ainsi gagner en sensibilité et en rapidité, l'ajout de milieu de culture a été testé.

Les échantillons ont été préparés selon le protocole décrit à l'exemple 6 ci-dessus. Les résultats présentés ici ont été obtenus avec *E. coli, E. faecalis et K Pneumoniae.*

Des concentrations croissantes de bactéries ont été ajoutées aux CP et incubées selon une cinétique allant de 0 à 16 heures en présence ou non de milieu trypticase de soja.

Les spectres montrent que les pics spécifiques de CP non contaminé n'occultent pas la mise en évidence de pics bactériens et la présence de pics supplémentaires provenant d'échantillons de CP infectés qui correspondent à la présence de protéines bactériennes supplémentaires.

Les résultats obtenus avec le logiciel BIOTYPER® sont le plus souvent faussement négatifs. Les scores d'identification obtenus avec le logiciel Biotyper avec inoculation de concentrations croissantes de *K Pneumoniae* de $10^2$ à $10^7$ ufc/ml en présence de milieu trypticase de soja sont inférieurs à 1.2 et ne permettent pas une identification. Même, après 6 heures de culture pour une concentration initiale à $10^7$ ufc/mL, le logiciel Biotype®r propose une identification incorrecte. En effet, il semble que, dans ce milieu complexe, l'association du spectre plaquettaire au spectre de la bactérie perturbe l'identification de germe par homologie spectrale.

En revanche, les analyses de spectre selon la nouvelle méthode exécutée par le logiciel dénommé logiciel IHU selon la présente invention, permet de détecter une pousse bactérienne en présence d'incubation de l'échantillon infecté avec ou sans milieu de culture comme rapporté à l'exemple 11 ci-après.

**Exemple 11 :** Analyse des CP après contamination bactérienne et ajout d'un milieu de culture et suivi de la pousse bactérienne avec le score d'identification du logiciel IHU.

On a appliqué la méthode d'analyse comparative de spectres décrite à l'exemple 4. A chaque fois les 2 spectres soustraits sont un spectre n°1 à t0=o (avec ajout de milieu et sans ajout de milieu) et un spectre n°2 après incubation de t2= 2h ou t6=6h ou t16=16h (avec ajout de milieu et respectivement sans ajout de milieu) notamment pour une concentration initiale à $10^2$ et $10^7$ ufc/ml avant incubation et après incubation en absence de milieu ou en présence de milieu trypticase de soja.

Sur les spectres obtenus après 16 heures d'incubation en présence de milieu trypticase de soja on a identifié des pics supplémentaires signant la croissance bactérienne détectable. Le tableau D ci-après rapporte les valeurs de score différentiel normalisé ou « score IHU » obtenues avec le logiciel IHU pour des spectres d'extrait obtenu à partir d'échantillons infectés avec inoculation de concentrations croissantes de K Pneumoniae de $10^2$ à $10^7$ ufc/ml après 0 à 16h d'incubation à 37°C en absence de milieu de culture Trypticase de soja.

Tableau D :

|      | $10^7$ | $10^4$ | $10^3$ | $10^2$ |
|------|--------|--------|--------|--------|
| tO   |        |        |        |        |
| t2   | 0,152  | 0,152  | 0,22   | 0,124  |
| t4   | 0,175  | 0,214  | 0,004  | 0,762  |
| t6   | 0,083  | 0,066  | 0,031  | 0,024  |
| t16  | 0,016  | 0,079  | 0,036  | 0,007  |

Le tableau E ci-après rapporte les valeurs de score différentiel normalisé ou « score IHU » obtenues avec le logiciel IHU pour des spectres d'extrait obtenu à partir d'échantillons infectés avec inoculation de concentrations croissantes de K Pneumoniae de $10^2$ à $10^7$ ufc/ml après 0 à 16h d'incubation à 37°C en présence de milieu de culture trypticase de soja.

Tableau E :

|      | $10^7$ | $10^4$ | $10^3$ | $10^2$ |
|------|--------|--------|--------|--------|
| t0   |        |        |        |        |
| t2   | 0,03   | 0,023  | 0,004  | 0,516  |
| t4   | 0,063  | 0,077  | 0,01   | 0,121  |
| t6   | 0,008  | 0,04   | 0,068  | 1,64   |
| t16  | 0,429  | 0,473  | 0,67   | 1,58   |

L'évolution du score IHU après 0 à 16 heures d'incubation à 37°C avec et sans bouillon triptycase soja permet de détecter une pousse bactérienne pour des concentrations initiales de $10^2$ ufc/ml à $10^7$ ufc/ml.

L'analyse des scores obtenus avec le logiciel IHU montre une augmentation significative du score IHU reflétant la contamination du CP par la bactérie Klebsiella Pneumoniae avec une augmentation importante du score IHU normalisé entre 6h et 16h pour des concentrations de $10^7$ ufc/ml et respectivement $10^2$ufc/ml de K.pneumoniae avec milieu trypticase de soja.

Des résultats positifs similaires d'augmentation du score IHU entre les extrait obtenus à partir d'échantillons ayant subi de 0 et 16 heures d'incubation à 37°C voire seulement 6h, et ce avec et sans milieu tripticase de soja ; pour des concentrations initiales de $10^2$ à $10^7$ UFC/ml, ont été obtenus pour toutes les bactéries testées , à savoir : *E. coli, Enterococcus faecalis, Pro videncia stuartii, Staphylococcus aureus, Staphylococcus epidermidis, Pseudomonas aeruginosa, Klebsiella oxytoca et Enterobacter cloacale.*

L'ajout du milieu favorise le développement de la bactérie et donc l'apparition de nouveau pics sur le spectre d'analyse. Ces pics supplémentaires signent la contamination du CP.

Le milieu de culture permet en outre quelques soit la concentration initiale de la bactérie d'obtenir après 16 heures d'incubation à 37°C une variation significative du score IHU. Cette augmentation du score IHU est le reflet des variations du spectre observées après incubation de l'échantillon en présence de trypticase soja favorisant la prolifération de la bactérie.

Les différents essais permettent de conclure qu'on peut paramétrer la méthode pour déterminer la présence d'une contamination de l'échantillon de concentré de plaquettes si le ratio entre le score différentiel normalisé (St), calculé sur un échantillon incubé pendant un temps t entre 0 et 24H par rapport à un échantillon de référence de concentré de plaquettes contaminé par la même bactérie à t=0 ou par rapport à un échantillon de de concentré de plaquettes non contaminé, et le score différentiel calculé à t=0 (So) par rapport à un échantillon de même nature non contaminé dépasse un seuil N2, N2 étant de préférence d'au moins 1.5.

## Revendications

**1.** Méthode de d'analyse d'échantillon biologique à analyser dans laquelle on réalise les étapes successives suivantes:

a) on réalise un spectre de masse de préférence du type MALDI-TOF du dit échantillon à analyser dénommé spectre à analyser, et

b) on réalise au moins un spectre de masse maldi-tof d'un échantillon de référence dénommé spectre de référence, et

c) on compare ledit spectre à analyser avec un dit spectre de référence essentiellement en calculant la soustraction entre les aires totales respectives du spectre à analyser et du spectre de référence.

**2.** Méthode selon la revendication 1, **caractérisée en ce qu'**à l'étape c), on réalise au moins les étapes successives suivantes :

i.1) on relève les intensités yi de respectivement chacun des points d'abscisse xi correspondant aux valeurs de rapport massique m/z de chaque spectre, et

i.2) pour chaque valeur de xi on calcule une intensité résultante yi3 par soustraction des intensités yi1 et yi2 respectives dudit spectre à analyser (yi1) et respectivement dudit spectre de référence (yi2) :yi3=yi2-yi1, et

i.3) on calcule la somme des intensités résultantes yi3 pour toutes les valeurs xi pour obtenir un score de caractérisation dénommé score $\text{différentiel} = \sum_{i=0}^{i=n} |yi3|$.

**3.** Méthode selon la revendication 2, **caractérisée en ce qu'**à l'étape c), on réalise au moins les étapes suivantes :

i.1) on normalise les intensités yi de respectivement chacun des points d'abscisse xi correspondant aux valeurs de rapport massique m/z de chaque spectre en divisant les intensités yi de chaque point d'abscisse xi (m/z) par l'intensité du pic de plus grande intensité, et

i.2) pour chaque valeur de xi on calcule une intensité résultante yi3 normalisée par soustraction des intensités normalisées respectives de deux spectres yi1 et yi2 :yi3=yi2-yi1, et

i.3) on calcule la somme des intensités résultantes normalisées yi3 pour toutes les valeurs xi pour obtenir un score de caractérisation dénommé $\text{score différentiel} = \sum_{i=0}^{i=n} |yi3|$, et

i.4) de préférence, on la normalise en la divisant par la somme des intensités pour toutes les valeurs xi de l'un des spectres, de préférence le spectre de plus petite somme des intensités pour toutes les valeurs xi, pour obtenir un score différentiel normalisé $= \sum_{i=0}^{i=n} |yi3| \sum_{i=0}^{i=n} yi1$

**4.** Méthode selon la revendication 2 ou 3, **caractérisée en ce qu'**à l'étape i.3), on réalise les étapes i.3a) et i.3b) de filtrage préalables avant l'étape 3c) suivantes :

i.3a) pour chaque valeur de xi, on enlève le bruit de fond en donnant la valeur d'intensité résultante yi3=0 si yi3 est inférieur à un seuil Sy1, Sy1 étant inférieur ou égal à 10% de la valeur yi max du pic de plus grande intensité, de préférence Sy1= 5% de la valeur yi max du pic de plus grande intensité, et

i.3b) on réalise un filtrage en donnant la valeur yi3=0 pour les valeurs de xi pour lesquels à xi' avec xi'-xi inférieur à Sx, Sx étant de préférence inférieur ou égal à 100Da de préférence inférieur à 10Da, l'intensité résultante yi3' est de signe opposée à l'intensité résultante yi3 et de valeur absolue d'au moins une valeur seuil relative de Sy2% celle de yi3, de préférence Sy2 étant d'au moins 70% de celle de yi3, notamment du fait d'un décalage des valeurs de xi correspondant aux intensités de deux pics d'un même composant des 2 spectres, et

i.3c) on calcule la somme des intensités restantes après filtrage yi3 pour toutes les valeurs xi pour obtenir un score de caractérisation dénommé $score\ différentiel = \sum_{i=0}^{i=n} |yi3|$, et

i.4) de préférence on la normalise en la divisant par la somme des intensités pour toutes les valeurs xi de l'un des spectres, de préférence le spectre de plus petite somme des intensités pour toutes les valeurs xi, pour obtenir un $score\ différentiel\ normalisé = \sum_{i=0}^{i=n} |yi3| / \sum_{i=0}^{i=n} yip$ avec p= 1 ou 2.

**5.** Méthode selon l'une des revendications 2 à 4, **caractérisée en ce que** le dit échantillon à analyser et le(s) dits échantillon(s) de référence sont issus de prélèvements matières biologiques, de préférence de fluide ou tissu animal ou humain, les dits échantillons de référence et échantillons à analyser pouvant être sains ou pathologiques.

**6.** Méthode selon l'une des revendications 2 à 5, **caractérisée en ce qu'**on paramètre les valeurs d'intensité et les dites valeurs seuils Sx, Sy1 et Sy2 de manière à ce que l'on détermine une valeur ou fourchette de valeurs de scores différentiels permettant de déterminer au moins l'identité de nature ou de qualité de l'échantillon à analyser et de l'échantillon de référence, et/ou des valeurs ou fourchette de valeurs de score différentiel de préférence permettant de déterminer le caractère pathologique de l'échantillon à analyser par rapport à un échantillon de même nature mais sain.

**7.** Méthode selon l'une des revendications 1 à 6, **caractérisée en ce qu'**à l'étape b) on réalise au moins un spectre de masse maldi-tof d'un échantillon de référence dénommé spectre de référence qui peut être :

b.1) un spectre de référence obtenu à partir d'un échantillon standard de même nature provenant d'une autre origine et/ou autre(s) individu(s) que ledit échantillon à analyser, ou

b.2) un spectre de référence d'un échantillon de même nature provenant de la même origine et/ou du même individu que le dit échantillon à analyser.

**8.** Méthode selon la revendication 7, **caractérisée en ce que** :

- à l'étape b), on réalise au moins un spectre de masse maldi-tof d'un échantillon de référence dénommé spectre de référence qui peut être :

b.1) un spectre de référence d'un échantillon standard sain de même tissu ou même fluide humain ou animal, ou

b.2) un spectre de référence d'un échantillon sain de même tissu ou même fluide du même individu ; et

- à l'étape c), on détermine si le dit échantillon à analyser est sain ou pathogène, de préférence tumoral et/ou on détermine le type de pathologie, de préférence le type de tumeur, selon la valeur de soustraction des aires des deux spectres, de préférence selon la valeur dudit score différentiel.

**9.** Méthode selon l'une des revendications 6 à 8, **caractérisée en ce que** ledit tissu est fragment d'organe solide, de préférence choisi parmi les fragments de tissu cutané, intestin, notamment oesophage, poumon, bronche, sein, organe de l'appareil uro-génital ou reproducteur masculin ou féminin , notamment prostate, testicule, vessie, utérus, et ganglions.

**10.** Méthode selon l'une des revendications 7 à 9, **caractérisée en ce que** :

- à l'étape b), on réalise au moins un spectre de référence établi sur au moins un échantillon de référence de fragment de même tissu sain standard ou provenant du même individu, et

- à l'étape c), on détermine si ledit fragment de tissu à analyser est un fragment de tissu de type non tumoral ou tumoral, de préférence appartenant à une desdites classes de tumeurs c.1 ou c.2 suivantes, selon la valeur de soustraction des aires des deux spectres comparés, de préférence selon la valeur dudit score différentiel :

    c.1- la classe des tumeurs cancéreuses, et
    c.2- la classe des tumeurs non cancéreuses.

**11.** Méthode selon la revendication 10, **caractérisée en ce qu'**à l'étape c), on détermine si ledit fragment de tissu à analyser est un fragment de tissu sain ou de type tumoral appartenant au moins à une desdites classes ou sous-classes de tumeurs suivantes , selon la valeur de soustraction des aires des deux spectres comparés, de préférence selon la valeur dudit score différentiel :

    c.1- la classe des tumeurs cancéreuses, et
    c.2.1- la sous-classe de tumeurs non cancéreuses non inflammatoires, et
    c.2.2- la sous-classe des tumeurs inflammatoires non cancéreuses.

**12.** Méthode selon l'une des revendications 9 à 11, **caractérisée en ce que** :

    - à l'étape a), on réalise un dit spectre d'un échantillon à analyser d'une suspension homogène de broyat de dit fragment d'organe comprenant un mélange pluricellulaire et/ou pluritissulaire de différents types de cellules entières non lysées, dilué de préférence dans de l'eau distillée, et
    - à l'étape b), on réalise au moins un spectre de référence d'au moins un échantillon de référence de suspension de broyat de respectivement au moins un fragment de référence brut de même tissu d'au moins un individu, le(s) dit échantillon(s) de référence étant préparé(s) de manière identique audit premier échantillon.

**13.** Méthode selon l'une des revendications 1 à 8, **caractérisée en ce que** :

    - le dit échantillon à analyser comprend un milieu complexe liquide ou solide contenant des différents types de protéines et/ou différents types de cellules, de préférence des cellules eucaryotes, de préférence des échantillons de fluide biologique humain ou animal contenant des cellules et des macromolécules biologiques, et
    - à l'étape a), on réalise un dit spectre d'un extrait protéique dudit d'un échantillon à analyser, et
    - à l'étape b), on réalise au moins un spectre de référence d'au moins un extrait protéique d'au moins un échantillon de référence de même nature standard ou provenant de la même origine ou même individu que ledit échantillon à analyser, le(s) dit échantillon(s) de référence étant préparé(s) de manière identique audit premier échantillon, et
    - à l'étape c), on détermine si le dit échantillon à analyser est sain ou pathogène et/ou on détermine le type de pathologie selon la valeur de soustraction des aires des deux spectres, de préférence selon la valeur dudit score différentiel.

**14.** Méthode selon la revendication 13, **caractérisée en ce que** ledit extrait protéique de dit échantillon à analyser et le dit échantillon de référence sont préparés par un procédé comprenant les étapes suivantes dans lesquelles :

    a.1) on réalise une lyse des cellules dudit échantillon, puis
    a.2) on réalise une extraction des protéines entières et solubilisées à partir d'un lysat dudit échantillon après culture, de l'étape a.1), et
    b) on réalise un spectre par spectrométrie de masse du type MALDI-TOF dudit extrait protéique obtenu à l'étape a.2), et
    c) on détermine si ledit échantillon à analyser est sain ou pathogène par comparaison du spectre obtenu à l'étape b) avec au moins un spectre de référence d'un extrait protéique de référence d'un échantillon standard de même nature que ledit échantillon à analyser ayant subi le même traitement de lyse et extraction des étapes a.1) et a.2).

**15.** Méthode selon l'une des revendications 3 à 14, **caractérisée en ce qu'**on met en oeuvre un programme d'ordinateur comportant au moins les instructions pour l'exécution des étapes i.1) à i.3) du procédé de traitement de spectres, de préférence un support d'enregistrement lisible par un ordinateur sur lequel est enregistré le dit programme d'ordinateur.

**Patentansprüche**

1. Verfahren zur Analyse einer zu analysierenden biologischen Probe, bei dem die folgenden aufeinanderfolgenden Schritte durchgeführt werden:

a) Erstellen eines Massenspektrums, vorzugsweise vom Typ MALDI-TOF, der zu analysierenden Probe, welches als zu analysierendes Spektrum bezeichnet wird, und

b) Erstellen wenigstens eines Maldi-Tof-Massenspektrums einer Referenzprobe, welches als Referenzspektrum bezeichnet wird, und

c) Vergleichen des zu analysierenden Spektrums mit einem Referenzspektrum im Wesentlichen durch Berechnen der Subtraktion zwischen den jeweiligen Gesamtflächen des zu analysierenden Spektrums und des Referenzspektrums.

2. Verfahren nach Anspruch 1, **dadurch gekennzeichnet, dass** bei Schritt c) wenigstens die folgenden aufeinanderfolgenden Schritte durchgeführt werden:

i.1) es werden die Intensitäten $y_i$ jeweils eines jeden der Abszissen-Punkte $x_i$, die den Masseverhältniswerten m/z eines jeden Spektrums entsprechen, aufgenommen, und

i.2) für einen jeden Wert von $x_i$ wird eine resultierende Intensität $y_{i3}$ durch Subtraktion der jeweiligen Intensitäten $y_{i1}$ und $y_{i2}$ des zu analysierenden Spektrums ($y_{i1}$) bzw. des Referenzspektrums ($y_{i2}$) : $y_{i3} = y_{i2} - y_{i1}$ berechnet, und

i.3) es wird die Summe der resultierenden Intensitäten $y_{i3}$ für alle Werte $x_i$ berechnet, um einen als Differenzscore = $\sum_{i=0}^{i=n} |y_{i3}|$ bezeichneten Charakterisierungsscore zu erhalten.

3. Verfahren nach Anspruch 2, **dadurch gekennzeichnet, dass** bei Schritt c) wenigstens die folgenden Schritte durchgeführt werden:

i.1) die Intensitäten $y_i$ jeweils eines jeden der Abszissen-Punkte $x_i$, die den Masseverhältniswerten m/z eines jeden Spektrums entsprechen, werden normalisiert, indem die Intensitäten $y_i$ eines jeden Abszissen-Punktes $x_i$ (m/z) durch die Intensität des Peaks mit höherer Intensität dividiert werden, und

i.2) für einen jeden Wert von $x_i$ wird eine normalisierte resultierende Intensität $y_{i3}$ durch Subtraktion der jeweiligen normalisierten Intensitäten von zwei Spektren $y_{i1}$ und $y_{i2}$ : $y_{i3} = y_{i2} - y_{i1}$ berechnet, und

i.3) es wird die Summe der normalisierten resultierenden Intensitäten $y_{i3}$ für alle Werte $x_i$ berechnet, um einen als Differenzscore = $\sum_{i=0}^{i=n} |y_{i3}|$ bezeichneten Charakterisierungsscore zu erhalten, und

i.4) vorzugsweise wird sie normalisiert, indem sie durch die Summe der Intensitäten für alle Werte $x_i$ von einem der Spektren dividiert wird, vorzugsweise dem Spektrum mit kleinerer Summe der Intensitäten für alle Werte $x_i$, um einen normalisierten Differenzscore = $\sum_{i=0}^{i=n}|y_{i3}| \sum_{i=0}^{i=n} |y_{i1}|$ zu erhalten.

4. Verfahren nach Anspruch 2 oder 3, **dadurch gekennzeichnet, dass** bei Schritt i.3) die folgenden vorhergehenden Filterschritte i.3a) und i.3b) vor Schritt 3c) durchgeführt werden:

i.3a) für jeden Wert von $x_i$ wird das Grundrauschen entfernt durch Vorgeben des Wertes der resultierenden Intensität $y_{i3} = 0$, wenn $y_{i3}$ kleiner als ein Schwellwert $S_{y1}$ ist, wobei $S_{y1}$ kleiner als oder gleich 10 % des Wertes $y_i$ max des Peaks mit höherer Intensität, vorzugsweise $S_{y1} = 5$ % des Wertes $y_i$ max des Peaks mit höherer Intensität ist, und

i.3b) es wird ein Filtern durchgeführt durch Vorgeben des Wertes $y_{i3} = 0$ für die Werte von $x_i$, bei denen bei $x_i'$, mit $x_i'-x_i$ kleiner als $S_x$, wobei $S_x$ vorzugsweise kleiner als oder gleich 100Da, vorzugsweise kleiner als 10Da, die resultierende Intensität $y_{i3}'$ ein zu der resultierenden Intensität $y_{i3}$ entgegengesetztes Vorzeichen und einen Absolutwert von wenigstens einem relativen Schwellwert $S_{y2}$% von demjenigen von $y_{i3}$ aufweist, wobei vorzugsweise $S_{y2}$ wenigstens 70 % von dem von $y_{i3}$ beträgt, insbesondere aufgrund einer Verschiebung der Werte von $x_i$, die den Intensitäten von zwei Peaks einer gleichen Komponente der 2 Spektren entsprechen, und

i.3c) es wird die Summe der nach Filtern verbleibenden Intensitäten $y_{i3}$ für alle Werte $x_i$ berechnet, um einen als Differenzscore = $\sum_{i=0}^{i=n} |y_{i3}|$ bezeichneten Charakterisierungsscore zu erhalten, und

i.4) vorzugsweise wird sie normalisiert, indem sie durch die Summe der Intensitäten für alle Werte $x_i$ von einem der Spektren dividiert wird, vorzugsweise dem Spektrum mit kleinerer Summe der Intensitäten für alle Werte xi, um einen normalisierten $\text{Differenzscore} = \sum_{i=0}^{i=n} |yi3| / \sum_{i=0}^{i=n} |yip|$, mit p = 1 oder 2, zu erhalten.

5. Verfahren nach einem der Ansprüche 2 bis 4, **dadurch gekennzeichnet, dass** die zu analysierende Probe und die Referenzprobe(n) aus Entnahmen biologischer Materialien, vorzugsweise von tierischer oder menschlicher Flüssigkeit oder tierischem oder menschlichem Gewebe stammen, wobei die Referenzproben und zu analysierenden Proben gesund oder pathologisch sein können.

6. Verfahren nach einem der Ansprüche 2 bis 5, **dadurch gekennzeichnet, dass** die Intensitätswerte und die Schwellwerte Sx, Sy1 und Sy2 parametriert werden, so dass ein Wert oder Wertebereich von Differenzscores bestimmt wird, der ermöglicht, wenigstens die Identität von Art oder Qualität der zu analysierenden Probe und der Referenzprobe zu bestimmen, und/oder vorzugsweise Werte oder Wertebereich eines Differenzscores bestimmt werden, die ermöglichen, den pathologischen Charakter der zu analysierenden Probe gegenüber einer Probe gleicher Art, die jedoch gesund ist, zu bestimmen.

7. Verfahren nach einem der Ansprüche 1 bis 6, **dadurch gekennzeichnet, dass** bei Schritt b) wenigstens ein als Referenzspektrum bezeichnetes Maldi-Tof-Massenspektrum einer Referenzprobe erstellt wird, das sein kann:

   b.1) ein Referenzspektrum, das anhand einer Standardprobe gleicher Art, die von einer anderen Quelle und/oder (einem) anderen Individuum/Individuen als die zu analysierende Probe stammt, erhalten wird, oder
   b.2) ein Referenzspektrum einer Probe gleicher Art, die von der gleichen Quelle und/oder von dem gleichen Individuum wie die zu analysierende Probe stammt.

8. Verfahren nach Anspruch 7, **dadurch gekennzeichnet, dass**:

   - bei Schritt b) wenigstens ein als Referenzspektrum bezeichnetes Maldi-Tof-Massenspektrum einer Referenzprobe erstellt wird, das sein kann:

     b.1) ein Referenzspektrum einer gesunden Standardprobe von gleichem menschlichen oder tierischen Gewebe oder gleicher menschlicher oder tierischer Flüssigkeit, oder
     b.2) ein Referenzspektrum einer gesunden Probe von gleichem Gewebe oder gleicher Flüssigkeit des gleichen Individuums, und

     - bei Schritt c) entsprechend dem Wert der Subtraktion der Flächen der beiden Spektren, vorzugsweise entsprechend dem Wert des Differenzscores, bestimmt wird, ob die zu analysierende Probe gesund oder pathogen, vorzugsweise tumoral ist, und/oder der Pathologietyp, vorzugsweise der Tumortyp, bestimmt wird.

9. Verfahren nach einem der Ansprüche 6 bis 8, **dadurch gekennzeichnet, dass** das Gewebe Fragment eines festen Organs ist, vorzugsweise ausgewählt aus den Fragmenten von Hautgewebe, Darm, insbesondere Speiseröhre, Lunge, Bronchie, Brust, Organ des Urogentialapparates oder männlichem oder weiblichem Fortpflanzungsorgan, insbesondere Prostata, Hoden, Harnblase, Gebärmutter und Lymphknoten.

10. Verfahren nach einem der Ansprüche 7 bis 9, **dadurch gekennzeichnet, dass**:

    - bei Schritt b) wenigstens ein Referenzspektrum, hergestellt an wenigstens einer gesunden Referenzprobe eines Fragments von gleichem Gewebe, Standard oder von dem gleichen Individuum stammend, erstellt wird, und
    - bei Schritt c) entsprechend dem Subtraktionswert der Flächen der beiden verglichenen Spektren, vorzugsweise entsprechend dem Wert des Differenzscores, bestimmt wird, ob das zu analysierende Gewebefragment ein Gewebefragment vom nicht tumoralen oder tumoralen Typ ist, der vorzugsweise zu einer der folgenden Tumorklassen c.1 oder c.2 gehört:

      c.1- der Klasse der kanzerösen Tumore und
      c.2- der Klasse der nicht kanzerösen Tumore.

11. Verfahren nach Anspruch 10, **dadurch gekennzeichnet, dass** bei Schritt c) entsprechend dem Subtraktionswert der Flächen der beiden verglichenen Spektren, vorzugsweise entsprechend dem Wert des Differenzscores, bestimmt wird, ob das zu analysierende Gewebefragment ein gesundes Gewebefragment oder Gewebefragment vom tumoralen Typ ist, der wenigstens zu einer der folgenden Tumorklassen oder-unterklassen gehört:

    c.1- der Klasse der kanzerösen Tumore und
    c.2.1- der Unterklasse von nicht kanzerösen, nicht inflammatorischen Tumoren, und
    c.2.2- der Unterklasse der inflammatorischen, nicht kanzerösen Tumore.

**12.** Verfahren nach einem der Ansprüche 9 bis 11, **dadurch gekennzeichnet, dass**:

    - bei Schritt a) ein Spektrum einer zu analysierenden Probe einer homogenen Suspension von Mahlgut eines Organfragments, umfassend eine mehrzellige und/oder Mehrgewebe-Mischung aus verschiedenen Typen von nicht lysierten ganzen Zellen, vorzugsweise in destilliertem Wasser verdünnt, erstellt wird, und
    - bei Schritt b) wenigstens ein Referenzspektrum wenigstens einer Referenzprobe einer Suspension von Mahlgut von jeweils wenigstens einem Rohreferenzfragment eines gleichen Gewebes wenigstens eines Individuums erstellt wird, wobei die Referenzprobe(n) identisch zu der ersten Probe hergestellt werden.

**13.** Verfahren nach einem der Ansprüche 1 bis 8, **dadurch gekennzeichnet, dass**:

    - die zu analysierende Probe ein komplexes flüssiges oder festes Medium umfasst, das verschiedene Typen von Proteinen und/oder verschiedene Typen von Zellen, vorzugsweise eukaryotische Zellen enthält, vorzugsweise Proben von menschlicher oder tierischer biologischer Flüssigkeit, die Zellen und biologische Makromoleküle enthält, und
    - bei Schritt a) ein Spektrum eines Proteinextraktes der zu analysierenden Probe erstellt wird, und
    - bei Schritt b) wenigstens ein Referenzspektrum wenigstens eines Proteinextraktes wenigstens einer Referenzprobe gleicher Art, Standard oder von der gleichen Quelle oder dem gleichen Individuum wie die zu analysierende Probe stammend, wobei die Referenzprobe(n) identisch zu der ersten Probe hergestellt werden, und
    - bei Schritt c) entsprechend dem Subtraktionswert der Flächen der beiden Spektren, vorzugsweise entsprechend dem Wert des Differenzscores, bestimmt wird, ob die zu analysierende Probe gesund oder pathogen ist und/oder der Pathologietyp bestimmt wird.

**14.** Verfahren nach Anspruch 13, **dadurch gekennzeichnet, dass** der Proteinextrakt einer zu analysierenden Probe und die Referenzprobe durch ein Verfahren hergestellt werden, das die folgenden Schritte umfasst, bei denen:

    a.1) eine Lyse der Zellen der Probe durchgeführt wird, dann
    a.2) eine Extraktion der ganzen und solubilisierten Proteine aus einem Lysat der Probe nach Kultivierung, des Schrittes a.1) vollzogen wird, und
    b) ein Spektrum durch Massenspektrometrie vom Typ MALDI-TOF des bei Schritt a.2) gewonnenen Extraktes erstellt wird, und
    c) bestimmt wird, ob die zu analysierende Probe gesund oder pathogen ist, indem das bei Schritt b) erhaltene Spektrum mit wenigstens einem Referenzspektrum eines Referenzproteinextraktes einer Standardprobe gleicher Art wie die zu analysierende Probe, welche der gleichen Lysebehandlung und Extraktion der Schritte a.1) und a.2) unterzogen wurde, verglichen wird.

**15.** Verfahren nach einem der Ansprüche 3 bis 14, **dadurch gekennzeichnet, dass** ein Computerprogramm verwendet wird, das wenigstens die Befehle für die Durchführung der Schritte i.1) bis i.3) des Verfahrens zur Verarbeitung von Spektren umfasst, vorzugsweise ein durch einen Computer lesbarer Aufzeichnungsträger, auf dem das Computerprogramm gespeichert ist.

**Claims**

**1.** A method of analyzing a biological sample in which the following successive steps are performed:

    a) taking a mass spectrum, preferably of the Maldi-Tof type, of said sample for analysis referred to as the "spectrum for analysis", and
    b) taking at least one Maldi-Tof mass spectrum of a reference sample referred to as the "reference spectrum"; and
    c) comparing said spectrum for analysis with a said reference spectrum essentially by calculating the difference between the respective total areas of the spectrum for analysis and the reference spectrum.

**2.** A method according to claim 1, **characterized in that**, in step c), at least the following successive steps are performed:

> i.1) taking the respective heights yi of each of the abscissa points xi corresponding to the mass ratio values m/z of each spectrum; and
> i.2) for each xi value calculating a resulting height yi3 by taking the difference between the heights yi1 and yi2 respectively of said spectrum for analysis (yi1) and respectively of said reference spectrum (yi2):

$$yi3 = yi2 - yi1$$

> and
> i.3) calculating the sum of the resulting heights yi3 for all of the xi values in order to obtain a characterization score referred to as the "differential" score:

$$\sum_{i=0}^{i=n} \left| yi3 \right| .$$

**3.** A method according to claim 2, **characterized in that**, in step c), at least the following steps are performed:

> i.1) normalizing the respective heights yi of each of the abscissa points xi corresponding to the m/z mass ratio values of each spectrum by dividing the heights yi of each abscissa point xi (m/z) by the height of the peak of greatest height; and
> i.2) for each value of xi, calculating a normalized resulting height yi3 by taking the difference between the respective normalized heights of two spectra yi1 and yi2:

$$yi3 = yi2 - yi1;$$

> and
> i.3) calculating the sum of the resulting heights yi3 for all of the xi values in order to obtain a characterization score referred to as the "differential" score:

$$\sum_{i=0}^{i=n} \left| yi3 \right| ;$$

> and
> i.4) preferably normalizing the sum by dividing it by the sum of the heights for all of the values xi of one of the spectra, preferably the spectrum having the smaller sum of heights for all the values xi, in order to obtain a normalized differential score:

$$\sum_{i=0}^{i=n} \left| yi3 \right| \sum_{l=0}^{i=n} yi1 .$$

**4.** A method according to claim 2 or claim 3, **characterized in that**, in step i.3), the following prior filtering steps i.3a) and i.3b) are performed before the step 3c):

> i.3a) for each value of xi, removing the background noise by giving the value yi3=0 to the resulting height if yi3 is less than a threshold Sy1, where Sy1 is less than or equal to 10% of the value yi max of the peak of greatest height, preferably Sy1 = 5% of the value of yi max of the peak of greatest height; and
> i.3b) filtering by giving the value yi3=0 for the values of xi for which at xi', with xi'-xi less than a threshold Sx, Sx preferably being less than or equal to 100Da, preferably less than 10Da, the resulting height yi3' is of sign opposite to the resulting height yi3 and of absolute value not less than a relative threshold value of Sy2% of the value of yi3, Sy2 preferably being at least 70% of the value of yi3; in particular as a result of an offset in the values of xi corresponding to the heights of the two peaks of a common component of the two spectra, and

i.3c) calculating the sum of the heights that remain after filtering yi3 for all of the xi values in order to obtain a characterization score referred to as the "differential" score:

$$\sum_{i=0}^{i=n} \left| yi3 \right| ;$$

and

i.4) preferably normalizing the sum by dividing it by the sum of the heights for all of the values xi of one of the spectra, preferably the spectrum having the smaller sum of heights for all the values xi, in order to obtain a normalized differential score: $\sum_{i=0}^{i=n} \left| yi3 \right| / \sum_{i=0}^{i=n} yip$ with p=1 or 2.

5. A method according to any one of claims 2 to 4, **characterized in that** said sample for analysis and said reference sample(s) are taken from biological matter, preferably animal or human fluid or tissue, it being possible for said reference samples and samples for analysis to be healthy or pathological.

6. A method according to any one of claims 2 to 5, **characterized in that** the height values and said threshold values Sx, Sy1, and Sy2 are set in such a manner as to determine a value or range of values for differential scores making it possible to determine at least the identity of the nature or quality of the sample for analysis and of the reference sample, and/or values or ranges of values for a differential score preferably making it possible to determine the pathological character of the sample for analysis relative to a sample of the same nature but that is healthy.

7. A method according to any one of claims 1 to 6, **characterized in that**, in step b) at least one Maldi-Tof mass spectrum is taken of a reference sample referred to as the "reference spectrum", which reference spectrum can be:

   b.1) a reference spectrum obtained from a standard sample of the same nature having an origin and/or coming from (an) individual(s) that is different to that of said sample for analysis; or
   b.2) a reference spectrum of a sample of the same nature of the same origin and/or coming from the same individual as said sample for analysis.

8. A method according to claim 7, **characterized in that**:

   • in step b), at least one Maldi-Tof mass spectrum is taken of a reference sample referred to as the "reference spectrum", which reference spectrum can be:

      b.1) a reference spectrum of a standard healthy sample of the same human or animal tissue or of the same human or animal fluid; or
      b.2) a reference spectrum of a standard healthy sample of the same tissue or of the same fluid from the same individual; and

   • in step c), it is determined whether said sample for analysis is healthy or pathogenic, preferably tumoral, and/or the type of pathology is determined, preferably the type of tumor, depending on the value of the difference between the areas of the two spectra, preferably depending on the value of said differential score.

9. A method according to any one of claims 6 to 8, **characterized in that** said tissue is a fragment of a solid organ, preferably selected from fragments of:

   cutaneous tissue; the intestine, in particular the esophagus; a lung, a bronchus; a breast; an organ of the male or female uro-genital or reproductive systems, in particular the prostate, a testicle, the bladder, the uterus, and ganglions.

10. A method according to any one of claims 7 to 9, **characterized in that**:

   • in step b), at least one reference spectrum is taken using at least one reference sample of a fragment of same standard healthy tissue or coming from the same individual; and
   • in step c), it is determined whether said fragment of tissue for analysis is a fragment of tissue of non-tumoral or tumoral type, preferably belonging to one of said following tumor classes c.1 or c.2, depending on the value of the difference between the areas of the two compared spectra, preferably depending on the value of said differential score:

      c.1- the class of cancerous tumors; and
      c.2- the class of non-cancerous tumors.

**11.** A method according to claim 10, **characterized in that**, in step c), it is determined whether said fragment of tissue for analysis is a fragment of healthy tissue or of tumoral type tissue, belonging to at least one of the following said tumor classes or sub-classes, depending on the value of the difference between the areas of the two spectra compared, preferably depending on the value of said differential score:

      c.1- the class of cancerous tumors; and
      c.2.1- the sub-class of non-cancerous, non-inflammatory tumors; and
      c.2.2- the sub-class of inflammatory non-cancerous tumors.

**12.** A method according to any one of claims 9 to 11, **characterized in that**:

    • in step a), a said spectrum is taken of a sample for analysis of a homogenate suspension of said organ fragment comprising a multicellular and/or multi-tissue mixture of different types of whole, non-lysed cells, preferably diluted in distilled water; and
    • in step b), at least one reference spectrum is taken of at least one homogenate suspension reference sample of respectively at least one untreated reference fragment of the same tissue of at least one individual, said reference sample(s) being prepared in identical manner to said first sample.

**13.** A method according to any one of claims 1 to 8, **characterized in that**:

    • said sample for analysis comprises a complex liquid or solid medium containing different types of proteins and/or different types of cells, preferably eucaryotic cells, preferably samples of human or animal biological fluid containing cells and biological macromolecules; and
    • in step a), a said spectrum of a protein extract of said sample for analysis is taken; and
    • in step b), at least one reference spectrum of at least one protein extract of at least one reference sample of the same standard nature or of the same origin or coming from the same individual as said sample for analysis is taken, said reference sample(s) being prepared in identical manner to said first sample; and
    • in step c), it is determined whether said sample for analysis is healthy or pathogenic, and/or the type of pathology is determined depending on the value of the difference between the areas of the two spectra, preferably depending on the value of said differential score.

**14.** A method according to claim 13, **characterized in that** said protein extract of said sample for analysis and of said reference sample are prepared by performing the following steps of:

    a.1) lysing the cells of said sample; then
    a.2) extracting whole and solubilized proteins from a lysate of said sample after culture from step a.1); and
    b) using a Maldi-Tof type mass spectrometer to take a spectrum of said protein extract obtained in step a.2); and
    c) determining whether said sample for analysis is healthy or pathogenic by comparing the spectrum obtained in step b) with at least one reference spectrum of a reference protein extract of a standard sample of the same nature as said sample for analysis having been subjected to the same lysis and extraction treatment of steps a.1) and a.2).

**15.** A method according to any one of claims 3 to 14, **characterized in that** a computer program is used including at least instructions for executing steps i.1) to i.3) of the spectrum processing method, preferably by using a computer-readable data medium on which said computer program is stored.

FIG.1

FIG.2A

FIG.2B

FIG.2C

FIG.3A

FIG.3B

FIG.3C

log score IHU,
Unités arbitraires

FIG.4

FIG.5A

FIG.5B

FIG.5C

FIG.6A

FIG.6B

FIG.6C

FIG.7

**EP 3 025 265 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2003027231 A

- WO 201115465 A

**Littérature non-brevet citée dans la description**

- **FOURNIER PE ; COUDERC C ; BUFFET S ; FLAUDROPS C ; RAOULT D.** Rapid and cost-effective identification of Bartonella species using mass spectrometry. *J Med Microbiol.,* 2009, vol. 58, 1154-1159